(19) Europäisches Patentamt
European Patent Office
Office européen des brevets

(11) **EP 4 167 249 A1**

(12) **EUROPEAN PATENT APPLICATION**
published in accordance with Art. 153(4) EPC

(43) Date of publication:
**19.04.2023 Bulletin 2023/16**

(21) Application number: **21826721.9**

(22) Date of filing: **16.06.2021**

(51) International Patent Classification (IPC):
*G16H 50/70* (2018.01)     *C12Q 1/6869* (2018.01)
*G16B 30/00* (2019.01)     *G16B 40/00* (2019.01)
*A61B 5/00* (2006.01)     *G16H 10/20* (2018.01)

(52) Cooperative Patent Classification (CPC):
**A61B 5/00; C12Q 1/6869; G16B 30/00;**
**G16B 40/00; G16H 10/20; G16H 50/70**

(86) International application number:
**PCT/KR2021/007567**

(87) International publication number:
**WO 2021/256860 (23.12.2021 Gazette 2021/51)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **16.06.2020  KR 20200073197**
**05.01.2021  KR 20210000946**

(71) Applicant: **CJ Bioscience, Inc.**
**Jung-gu**
**Seoul 04527 (KR)**

(72) Inventors:
• **MIN, Ui Gi**
**Seoul 06643 (KR)**
• **OH, Hyeon Seok**
**Seoul 08802 (KR)**
• **KIM, Nam Il**
**Seoul 02434 (KR)**
• **LIM, Jeong Min**
**Seoul 05241 (KR)**
• **KIM, Eun Hye**
**Incheon 21986 (KR)**

(74) Representative: **Regimbeau**
**20, rue de Chazelles**
**75847 Paris Cedex 17 (FR)**

Remarks:
The complete document including Reference
Table(s) and the Sequence Listing(s) can be
downloaded from the EPO website

(54) **APPARATUS FOR DETERMINING INTESTINAL MICROBIOME INDEX, METHOD THEREFOR, AND RECORDING MEDIUM RECORDING INSTRUCTION THEREFOR**

(57)     The present disclosure proposes an apparatus for determining an intestinal microbiome index. The apparatus according to the present disclosure: obtains test information about a biological sample of a subject from a test apparatus; determines, based on the test information, first information about the similarity of intestinal microflora, second information about a proportion of harmful intestinal microflora, third information about a proportion of beneficial intestinal microflora, and/or fourth information about the diversity of intestinal microflora; and determines an intestinal microbiome index indicating a state of intestinal microbiome of the subject based on the determined information.

FIG. 1

**Description**

TECHNICAL FIELD

The present disclosure relates to a technique for determining the gut microbiome index.

BACKGROUND

A large number of microbes are commensal within the human body, and these microbes are found in the respiratory organs, mouth, skin, and the like but most are present in digestive organs including the large intestine. The commensal microbes form communities without causing disease, and these communities are called normal flora. The normal flora plays a role in preventing pathogenic microbes harmful to humans from settling in the body. In particular, hundreds of species of microbes live in complicated ecosystems within the gut, and thus the maintenance of normal flora is important for a person to keep a healthy state. The process of keeping a balanced state through the co-working of the normal flora and the gut immune system is called homeostasis. However, when the normal flora becomes imbalanced by external causes or the ratio between the normal flora and the harmful pathogenic microbes is changed to be out of the normal range, this state is called dysbiosis. Recent studies have revealed that an imbalance occurring in the gut is associated with various types of symptoms or various diseases and is typically associated with inflammatory bowel disease, metabolic syndromes, obesity, diabetes, cardiovascular diseases, and the like.

Therefore, it is important to accurately understand the state of gut flora in order to determine an individual's health state or the risk of the individual's disease. The developments of next-generation sequencing (NGS) technology and bioinformatics enabling the analysis of a wide range of genomic information can make a quick analysis of the gut flora. The gut microbiome refers to collective genetic information of gut microbial communities thus analyzed, and the gut microbiome can be utilized to understand the state of the gut flora.

However, the state of the gut flora cannot be simply derived through the simplification based on only a few microbial species but needs to be understood through ecological approach. The reason is that the gut flora itself is composed of hundreds of microbes forming complicated ecosystems. However, conventional diagnostic methods are restricted since the methods simply depend on the known functional characteristics of microbes detected in the gut or only a subject's health records are merely used. Moreover, an inaccurate diagnosis on the state of the gut microbiome decrease the efficiency of attempts to modulate the gut microbiome (e.g., ingesting probiotics).

Therefore, there is a need for a method for diagnosing the health condition of a subject by comprehensively considering ecological indexes of the gut microbiome. In addition, there is a need for a method for sub-classifying the enterotype on the basis of the gut microbiome of a subject and modulating the gut microbiome suitable for the enterotype of the subject.

SUMMARY

Various embodiments of the present disclosure provide a technique for determining the gut microbiome index.

In accordance with one aspect of the present disclosure, there is a provided apparatus for determining a gut microbiome index. The apparatus according to one aspect of the present disclosure includes: a communication circuit; at least one processor; and at least one memory configured to store instructions that cause at least one processor to perform an operation when the instructions are executed by at least one processor, wherein at least one processor is configured to: acquire test information about a bio-sample of a subject from at least one test apparatus, by using the communication circuit; based on the test information, determine first information about microbial similarity between a predetermined reference sample and the bio-sample, second information about proportions of harmful gut microbes of the subject, third information about proportions of beneficial gut microbes of the subject, and fourth information about gut microbial diversity of the subject; and based on the first information, the second information, the third information, and the fourth information, indicate a state of the gut microbiome of the subject.

At least one processor is configured to transmit information indicating the gut microbiome index to a device of the subject by using the communication circuit.

At least one memory is configured to further store information about the reference sample, and at least one processor is configured to: based on the test information and the information about the reference sample, determine similarity between the distribution of the presence proportion of each microbial species in the bio-sample and the distribution of the presence proportion of each microbial species in the reference sample; and based on the similarity, determine the first information.

At least one processor is configured to: based on the test information, determine, relative to predetermined commensal microbial species and harmful microbial species in the bio-sample, presence proportions of the harmful microbial species; and based on the presence proportions, determine the second information.

At least one processor is configured to: based on the test information, determine presence proportions of predetermined beneficial microbial species in the bio-sample; and based on the presence proportions, determine the third information.

At least one processor is configured to: based on the test information, determine the number of all the microbial species in the bio-sample and the degree to which the distribution of the presence proportion of each of all the microbial species are even; and based on the number of all the microbial species and the evenness, determine the fourth information.

At least one processor is configured to determine the gut microbiome index by applying predetermined weights to the first information, the second information, the third information, and the fourth information, respectively.

In accordance with one aspect of the present disclosure, there is provided a method for determining a gut microbiome index. The method according to one aspect of the present disclosure is performed by an apparatus including at least one processor and at least one memory storing instructions executed by at least one processor. The method for determining a gut microbiome index includes: acquiring, by at least one processor, test information about a bio-sample of a subject from at least one test apparatus; based on the test information, determining, by at least one processor, first information about microbial similarity between a predetermined reference sample and the bio-sample, second information about proportions of harmful gut microbes of the subject, third information about proportions of beneficial gut microbes of the subject, and fourth information about gut microbial diversity of the subject; and based on the first information, the second information, the third information, and the fourth information, determining, by at least one processor, a gut microbiome index indicating a state of the gut microbiome of the subject.

In accordance with one aspect of the present disclosure, there is provided a non-transitory computer-readable recording medium storing instructions for determining a gut microbiome index. The instructions stored in the recording medium according to one aspect of the present disclosure are instructions to be executed on a computer, and the instructions, when executed by at least one processor, cause at least one processor to: based on test information about a bio-sample of a subject, acquired from at least one test apparatus, determine first information about microbial similarity between a predetermined reference sample and the bio-sample, second information about proportions of harmful gut microbes of the subject, third information about proportions of beneficial gut microbes of the subject, and fourth information about gut microbial diversity of the subject; and based on the first information, the second information, the third information, and the fourth information, determine a gut microbiome index indicating a state of the gut microbiome of the subject.

In accordance with one aspect of the present disclosure, there is provided an apparatus for determining an enterotype. The apparatus according to one aspect of the present disclosure includes: a communication circuit; at least one processor; and at least one memory configured to store instructions that cause at least one processor to perform an operation when the instructions are executed by at least one processor, wherein at least one processor is configured to: acquire test information about a bio-sample of a subject from at least one test apparatus, by using the communication circuit; determine an enterotype of the subject as a first type, based on: a level of an inflammation-specific biomarker relative to the bio-sample in the test information; and/or a gut microbiome index indicating a state of the gut microbiome of the subject and determined based on the test information; and acquire gut management information related to the enterotype from at least one memory, wherein the gut management information indicates gut microbiome regulating suggestions for the subject with the enterotype.

At least one processor is configured to transmit information indicating the enterotype and the gut management information to a device of the subject, by using the communication circuit.

The inflammation-specific biomarker is at least one microbiome selected from the phylum *Proteobacteria* microbes and the genus *Fusobacterium* microbes.

At least one processor is configured to determine the enterotype as the first type if the presence proportion of the phylum Proteobacteria microbes relative to the bio-sample exceeds 10% or the presence proportion of the genus *Fusobacterium* microbes relative to the bio-sample exceeds 1%.

At least one processor is configured to determine the enterotype as the first type if the level of the inflammation-specific biomarker relative to the bio-sample satisfies a predetermined first standard and/or the gut microbiome index satisfies a predetermined second standard.

At least one processor is configured to: based on the test information, determine first information about microbial similarity between a predetermined reference sample and the bio-sample, second information about proportions of harmful gut microbes of the subject, third information about proportions of beneficial gut microbes of the subject, and fourth information about gut microbial diversity of the subject; and based on the first information, the second information, the third information, and the fourth information, determine the gut microbiome index.

At least one processor is configured to determine the gut microbiome index by applying predetermined weights to the first information, the second information, the third information, and the fourth information, respectively.

At least one processor is configured to transmit information indicating the the enterotype, the gut management information, and information indicating the gut microbiome index to a device of the subject by using the communication circuit.

At least one processor is configured to, when the enterotype of the subject is not determined as the first type, determine the enterotype as the second type if the presence proportion of the genus Prevotella microbes relative to the bio-sample exceeds 3%, and determine the enterotype as the third type if does not exceed 3%.

The gut microbiome regulating suggestions of the gut management information includes at least one selected from ingesting specific probiotics corresponding to the enterotype, ingesting a specific food, and applying a specific life habit.

The gut microbiome regulating suggestions of the gut management information have priority according to the degree to which each of the suggestions changes the gut microbiome index of the subject with the enterotype.

The gut microbiome regulating suggestions includes: (i) suggesting, to a subject classified as the first type, probiotics containing at least one species of microbes including at least one *Lactobacillus paracasei,* at least one *Lactobacillus fermentum,* at least one *Lactobacillus plantarum* group, and/or at least one *Lactococcus lactis* group; (ii) suggesting, to a subject classified as the second type 1020, probiotics containing at least one species of microbes including at least one *Lactobacillus fermentum* and/or at least one *Lactococcus lactis* group; or (iii) suggesting, to a subject classified as the third type 1030, probiotics containing at least one species of microbes including at least one at least one *Lactobacillus fermentum,* at least one *Lactobacillus plantarum* group, and/or at least one *Lactococcus lactis* group.

In one embodiment, at least one *Lactobacillusparacasei* includes *Lactobacillusparacasei subsp. paracasei, Lactobacillus paracasei subsp. tolerance,* or *Lactobacillus paracasei subsp. tolerance* HM0866 (accession number: KCTC14409BP).

In one embodiment, at least one *Lactobacillus fermentum* includes *Lactobacillus fermentum* HM0740 (accession number: KCTC14406BP).

At least one *Lactobacillus plantarum* group includes at least one selected from the group consisting of *Lactobacillus plantarum, Lactobacillus pentosus, Lactobacillus paraplantarum, Lactobacillus fabifermentans, Lactobacillus xiangfangensis, Lactobacillus herbarum, Lactobacillus modestisalitolerans,* and subspecies thereof.

In one embodiment, the *Lactobacillus plantarum* includes *Lactobacillus plantarum subsp. plantarum, Lactobacillus plantarum subsp. argentoratensis,* or *Lactobacillus plantarum subsp. plantarum* HM0782 (accession number: KCTC14407BP).

At least one *Lactococcus lactis* group includes at least one selected from the group consisting of *Lactococcus lactis subsp. lactis, Lactococcus lactis subsp. hordniae, Lactococcus lactis subsp. cremoris, Lactococcus lactis subsp. tructae,* and *Lactococcus lactis subsp. lactis* HM0850 (accession number: KCTC14408BP).

In one embodiment, at least one *Lactobacillusparacasei* includes *Lactobacillusparacasei subsp. tolerance* HM0866 (accession number: KCTC14409BP), at least one *Lactobacillus fermentum* includes *Lactobacillus fermentum* HM0740 (accession number: KCTC14406BP), at least one *Lactobacillus plantarum* includes *Lactobacillus plantarum subsp. plantarum* HM0782 (accession number: KCTC14407BP), and at least one *Lactococcus lactis* group includes *Lactococcus lactis subsp. lactis* HM0850 (accession number: KCTC14408BP).

In accordance with one aspect of the present disclosure, there is provided a method for determining an enterotype. The method according to an aspect of the present disclosure is a method performed by an apparatus including at least one processor and at least one memory storing instructions executed by at least one processor. The method for determining an enterotype includes: acquiring, by at least one processor, test information about a bio-sample of a subject from at least one test apparatus; determining, by at least one processor, the enterotype of the subject as a first type, based on the level of an inflammation-specific biomarker relative to the bio-sample in the test information; and acquiring, by at least one processor, gut management information related to the enterotype from at least one memory, wherein the gut management information indicates gut microbiome regulating suggestions for the subject with the enterotype.

In accordance with one aspect of the present disclosure, there is provided a non-transitory computer-readable recording medium storing instructions for determining an enterotype. The instructions recorded in the recording medium according to one aspect of the present disclosure are instructions that, when executed by at least one processor, cause at least one processor to: determine the enterotype of a subject as a first type, based on the level of an inflammation-specific biomarker relative to the bio-sample, in the test information about a bio-sample of the subject acquired from at least one test apparatus; and acquire gut management information related to the enterotype from at least one memory, wherein the gut management information indicates gut microbiome regulating suggestions for the subject with the enterotype.

According to the present disclosure in some embodiments, the health condition of a subject can be diagnosed by comprehensively considering various ecological indexes of the gut microbiome of the subject.

According to the present disclosure in some embodiments, the enterotype of a subject can be sub-classified on the basis of the gut microbiome of the subject.

According to the present disclosure in some embodiments, gut microbiome regulating suggestions suited to the enterotype of a subject can be provided.

BRIEF DESCRIPTION OF DRAWINGS

FIG. 1 is a diagram showing an operation of an apparatus according to one embodiment of the present disclosure.
FIG. 2 is a block diagram showing an apparatus according to one embodiment of the present disclosure.
FIG. 3 is a diagram showing microbial similarity-related indexes according to one embodiment of the present disclosure.
FIG. 4 is a diagram showing harmful microbe-related indexes according to one embodiment of the present disclosure.
FIG. 5 is a diagram showing beneficent microbe-related indexes according to one embodiment of the present disclosure.
FIG. 6 is a diagram showing microbial diversity-related indexes according to one embodiment of the present disclosure.
FIG. 7 is a diagram showing a function for determining a gut microbiome index according to one embodiment of the present disclosure.
FIG. 8 is a diagram showing the test results of accuracy of gut the microbiome index according to one embodiment of the present disclosure.
FIG. 9 is a diagram showing the test results of accuracy of gut the microbiome index according to one embodiment of the present disclosure.
FIG. 10 is a diagram showing an operation of an apparatus according to one embodiment of the present disclosure.
FIG. 11 is a diagram showing gut management information according to one embodiment of the present disclosure.
FIG. 12 is a diagram showing a gut microbiome index determining method according to one embodiment of the present disclosure.
FIG. 13 is a diagram showing an enterotype determining method according to one embodiment of the present disclosure.
FIG. 14 is a diagram showing average changes in gut microbiome index before and after ingestion of strains according to one embodiment of the present disclosure.

DETAILED DESCRIPTION

The various embodiments described herein are exemplified for the purpose of clearly describing the technical idea of the present disclosure, and are not intended to limit the technical idea of the present disclosure to specific embodiments. The technical idea of the present disclosure includes various modifications, equivalents, alternatives of each of the embodiments described in this document, and embodiments selectively combined from all or part of the respective embodiments. In addition, the scope of the technical idea of the present disclosure is not limited to various embodiments or detailed descriptions thereof presented below.
The terms used herein, including technical or scientific terms, have meanings that are generally understood by a person having ordinary knowledge in the art to which the present disclosure pertains, unless otherwise specified.
The expressions such as "include", "provided with", and "have" mean the presence of subject features (e.g., functions, operations, components, etc.) and do not exclude the presence of other additional features. That is, such expressions should be understood as open-ended terms that imply the possibility of including other embodiments.
A singular expression can include a meaning of plurality, unless otherwise mentioned, and the same is applied to a singular expression stated in the claims.
The terms "first", "second", etc. used herein are used to identify a plurality of components from one another in referring to plural same objects unless otherwise indicated in the context, and are not intended to limit the order or importance of the relevant components.
The expressions used herein, "A, B and C," "A, B or C," "A, B and/or C," "at least one of A, B, and C," "at least one of A, B, or C," "at least one of A, B, and/or C," "at least one selected from A, B, and C", "at least one selected from A, B, or C", "at least one selected from A, B, and/C", and the like are used to refer to each listed item or any possible combination of the listed items is provided. For example, the expression "at least one of A and B" refers to all of (1) A, (2) at least one of A, (3) B, (4) at least one of B, (5) at least one of A and at least one of B, (6) B and at least one of A, (7) A and at least one of B, and (8) both of A and B.
The expression "on the basis of" used herein is used to describe one or more factors that influences a decision, an action of judgment or an operation described in a phrase or sentence including the relevant expression, and this expression does not exclude additional factor influencing the decision, the action of judgment or the operation.
As used herein, the expression that a certain component (e.g., a first component) is "connected" to another component (e.g., a second component) means that the certain component is connected to another component either directly or via a new different component (e.g., a third component).

As used herein, the expression "configured to" has a meaning such "set to", "having the ability to", "modified to", "made to", "capable of", or the like depending on the context. The expression is not limited to the meaning of "specially designed for hardware." For example, a processor configured to perform a specific operation means a generic-purpose processor capable of performing the specific operation by executing software.

The expression "unit" used herein refers to a software component or hardware component, such as a field-programmable gate array (FPGA) and an application specific integrated circuit (ASIC). However, the "unit" is not limited to software and hardware. The "unit" is configured to be an addressable storage medium or is configured to run on one or more processors. For example, the "unit" includes components, such as software components, object-oriented software components, class components, and task components, as well as processors, functions, attributes, procedures, subroutines, segments of program codes, drivers, firmware, micro-codes, circuits, data, databases, data structures, tables, arrays, and variables.

The description of dimensions, numerical values, and ranges thereof used herein, unless otherwise specified in context, are not limited to only the corresponding dimensions, numerical values, and ranges thereof, and means equivalent ranges including these.

Hereinafter, various embodiments of the present disclosure will be described in detail with reference to the accompanying drawings. In the accompanying drawings and the descriptions of the drawings, identical or substantially equivalent elements are given the same reference numerals. In the following description of the various embodiments, a description of the same or corresponding components are omitted, but this does not mean that the components are not included in the embodiment.

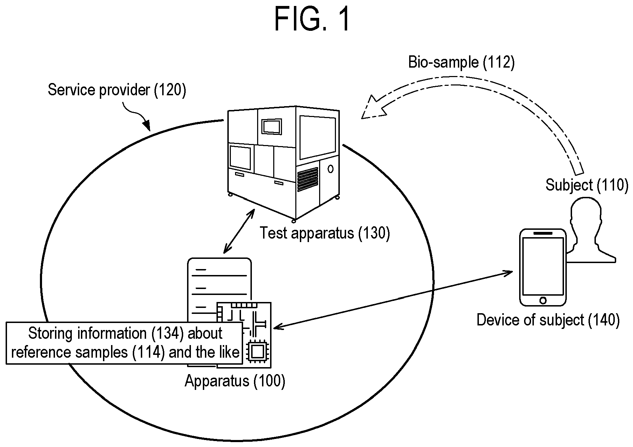

FIG. 1 is a diagram showing an operation of an apparatus 100 according to one embodiment of the present disclosure. The apparatus 100 according to one embodiment of the present disclosure determines the gut microbiome index of a subject by comprehensively considering various factors.

A subject 110 and a service provider 120 exchange a sample or information to determine the gut microbiome index of the subject 110. In the present disclosure, the subject 110 is a person (natural person) to be diagnosed for his or her gut microbiome. In the present disclosure, the service provider 120 diagnoses the gut microbiome of the subject 110 by using a bio-sample 112 provided from the subject 110. The service provider 120 is at least one entity that operates at least one test apparatus 130 and/or the apparatus 100 for determining the gut microbiome index. In the present disclosure, the microbiome refers to a microbial community, an entire microbial population, or a microbial ecosystem, wherein microbes are commensal and live in a given environment in the human body. The gut microbiome means a microbial community that inhabit the human gut and are commensal with humans. In some cases, the microbiome means the entire genetic information of a microbial community. In the present disclosure, the gut microbiome index is an index indicating the state (e.g., ecological balance, imbalance, etc.) of the gut microbiome of the subject 110.

The subject 110 provides the bio-sample 112 to the service provider 120. In the present disclosure, the bio sample is a human-derived material obtained from the body of the subject 110. In one embodiment, the bio-sample is a stool sample of the subject 110. In one embodiment, the bio-sample is tissue, cells, blood, a body fluid, serum, plasma, chromosome, protein, red blood cells, body hair, urine, saliva, or sweat.

At least one test apparatus (hereinafter, "test apparatus") 130 tests the bio-sample 112 by various methods. In one embodiment, the test apparatus 130 performs, on the bio-sample 112, molecular biological tests, such as next generation sequencing (NGS) and polymerase chain reaction (PCR), biomarker tests, genetic tests, and the like. The information resulting from various tests performed on the bio-sample 112 of the subject 110 by the test apparatus 130 (hereinafter, "test information") is transmitted to the apparatus 100. In one embodiment, the test information is various types of raw data with respect to the bio-sample 112 or data that has been subjected to predetermined processing by the test apparatus 130. In one embodiment, the apparatus 100 directly receives the test information 132 from the test apparatus 130. That is, the apparatus 100 receives test information 132 by directly communicating with the test apparatus 130 according to various wired/wireless communication methods. In one embodiment, the apparatus 100 indirectly receives the test information 132 from the test apparatus 130. That is, once the test apparatus 130 prepares the test information 132, a server or another recording medium stores corresponding test information 132, and then the apparatus 100 communicates with the server to receive the test information 132 or receive the test information 132 from the corresponding recording medium. Alternatively, the apparatus 100 receives the test information 132 in a manner in which the service provider 120 inputs the test information 132 to the apparatus 100.

In one embodiment, the test information 132 of the subject 110 includes information obtained by performing steps of obtaining genomic DNA of gut microbes from the bio-sample 112 of the subject, obtaining 16S rRNA genetic information of the gut microbes from gut microbial DNA, and analyzing the obtained 16S rRNA genetic information of the gut microbes to obtain microbial species that are distinguished at the level of sepcies in the gut microbial community of the subject 110 and presence proportions of the microbial species. On the basis of the test information 132, first information to fourth information of the subject are determined. In addition, the 16S rRNA genetic information is analyzed using a next generation sequencing (NGS) platform.

In one embodiment, the analyzing of the 16S rRNA genetic information of the gut microbes includes performing PCR using a pramier set capable of specifically amplifying variable regions of 16S rRNA, preferably performing PCR using a primer set capable of specifically amplifying V3 or V4 region of 16S rRNA, and more preferably, performing PCR using universal primers having the following sequences to generate an amplicon, and exemplary sequences of the universal primers are as below.

Forward universal primer (SEQ ID NO: 75): 5'-CCTACGGGNGGCWGCAG-3'
Reverse universal primer (SEQ ID NO: 76): 5'-GACTACHVGGGTATCTAATCC-3'

In one embodiment, the obtaining of microbial species that are distinguished at the level of species in the gut microbial community and presence proportions of these microbial species is performed by a step of analyzing bacterial community information at levels from phylum to species with the aid of the 16S ribosomal RNA gene sequence database (EzTaxon) of standard strains and non-cultured microbes and the EasyBioCloud analysis system (http://www.ezbiocloud.com) on the basis of thousands of gene sequences generated from one sample by the next-generation sequencing (NSG) technique. When products of the next-generation sequencing technique are identical, the method for microbiome information analysis is not limited to EzTaxon and the EasyBioCloud analysis system. The database used for identifying and classifying microbes is appropriately selected, as necessary, by a person skilled in the art. For example, the database is at least one selected from the group consisting of EzBioCloud, SILVA, RDP, and Greengene, but is not limited thereto.

In one embodiment, the scale of the microbial community is expressed as a relative presence (%) of a specific microbiome in the entire gut microflora. The relative presence (%) of the microbial community is a percentage of 16S rRNA read frequencies of the specific microbe in all sequencing reads.

In one embodiment, the apparatus 100 acquires the test information 132 from the test apparatus 130 and determine various information related to the gut microbiome of the subject 110 on the basis of the test information 132. The gut microbiome includes a plurality of microbes. One or two microbial species do not cause health problems or disease, but all the microbes in the gut microbiome play each role or overall roles, thereby contributing to gut ecological balances. Therefore, in order to diagnose whether the gut microbiome is in an ecological imbalance state, various factors need to be comprehensively considered in an ecological aspect. Accordingly, the apparatus 100 determines various information related to the gut microbiome of the subject 110. The factors that influence the gut ecological balance/imbalance include gut microbial similarity, proportions of harmful gut microbes, proportions of beneficial gut microbes, and/or gut microbial diversity. The gut microbial similarity refers to the degree to which the gut microbiome of the subject 110 is similar to the gut microbiome of a healthy person (hereinafter, "healthy person"). The healthy person refers to a person who is classified as being in a normal or balanced gut microbiome state according to predetermined criteria.

In one embodiment, the apparatus 100 determines information about gut microbial similarity (hereinafter, "first information"). That is, the first information is information indicating how similar the gut microbiome of the subject 110 is to the gut microbiome of a healthy person. The apparatus 100 stores information 134 about a reference sample. The information 134 about a reference sample is information obtained by performing the aforementioned various tests on a predetermined reference sample 114. The reference sample 114 is a bio-sample from a healthy person classified according to predetermined criteria. The apparatus 100 determines the aforementioned first information, on the basis of microbial similarity between the bio-sample 112 and the reference sample 114.

In one embodiment, the apparatus 100 determines information about the proportions of harmful gut microbes of the subject 110 (hereinafter, "second information"). That is, the second information includes information about the presence proportions or absolute amounts (abundance) of harmful gut microbes of the subject 110, information about the diversity of harmful gut microbial species, and/or information about the evenness of the presence proportion of each harmful microbial species. In one embodiment, the second information includes information about the ratio of harmful gut microbes to beneficial gut microbes in the subject 110. In one embodiment, the apparatus 100 stores information specifying which microbes in the microbes are harmful or beneficial.

In one embodiment, the apparatus 100 determines information about the proportions of beneficial gut microbes of the subject 110 (hereinafter, "third information"). That is, the third information includes information about the presence proportions or absolute amounts (abundance) of beneficial gut microbes of the subject 110, information about the diversity of beneficial gut microbial species, and/or information about the evenness of the presence proportion of each beneficial microbial species. In one embodiment, the third information includes information about the ratio of beneficial gut microbes to harmful gut microbes in the subject 110.

In one embodiment, the apparatus 100 determines information about gut microbial diversity of the subject 110 (hereinafter, "fourth information"). That is, the fourth information is information about how various microbial species are distributed in the gut of the subject 110.

The apparatus 100 determines the gut microbiome index by comprehensively considering various ecological information about the gut microbiome of the subject 110. In one embodiment, the gut microbiome index is used to distinguish between a healthy person and a diseased person (hereinafter, "diseased person"). A diseased person refers to a person who is classified as being in an imbalanced gut microbiome state according to predetermined criteria. In one embodiment, the apparatus 100 determines the gut microbiome index of the subject 110 on the basis of the first information, second information, third information, and/or fourth information. In one embodiment, the apparatus 100 determines the gut microbiome index by inputting the first information, second information, third information, and/or fourth information into a predetermined function. In one embodiment, the apparatus 100 applies predetermined weights to the first information, second information, third information, and/or fourth information in the determination of the gut microbiome index.

Information 136 indicating the gut microbiome index determined by the apparatus 100 is provided to the subject 110 through various methods. In one embodiment, the apparatus 100 transmits the information 136 indicating the gut microbiome index to the device 140 of the subject by an electronic information transmission method. In the present disclosure, the device 140 of the subject includes various forms of devices. For example, the device 140 of the subject is a portable communication device (e.g., a smartphone), a computer device (e.g., a tablet PC or a laptop), a portable multimedia device, a wearable device, or a device according to a combination of the aforementioned devices. In one embodiment, the service provider 120 transmits the information 136 indicating the gut microbiome index to the subject 110 by a non-electronic information transmission method (e.g., mail, face-to-face delivery by a medical person, etc.).

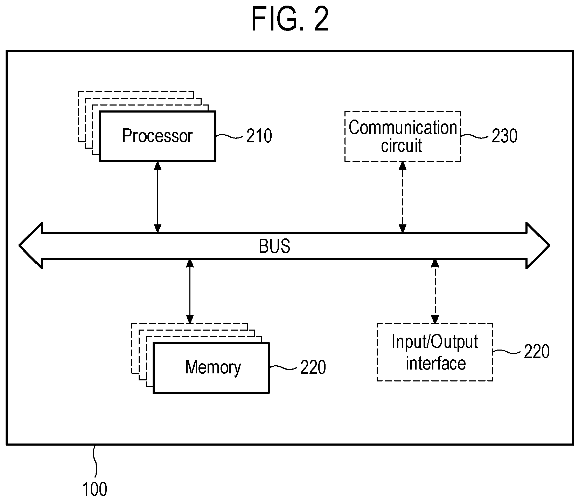

FIG. 2 is a block diagram showing a device 100 according to one embodiment of the present disclosure. In one embodiment, the apparatus 100 includes at least one processor 210 and/or at least one memory 220 as a component. In one embodiment, at least one of the components of the apparatus 100 are omitted, or another component is added to the apparatus 100. In one embodiment, additionally or alternatively, some components are integrated or are implemented as a singular entity or plural entities. In the present disclosure, at least one processor 210 is expressed as a processor 210. The expression processor 210 refers to a set of one or more processors, unless clearly expressed otherwise in context. In the present disclosure, at least one memory 220 is expressed as a processor 210. The expression memory 220 refers to a set of one or more memories, unless clearly expressed otherwise in context. In one embodiment, at least some of internal and external components of the apparatus 100 are connected to each other via a bus, a general purpose input/output (GPIO), a serial peripheral interface (SPI), a mobile industry processor interface (MIPI), or the like to exchange information (data, signals, etc.).

The processor 210 controls at least one component of the apparatus 100 connected to the processor 210 by running software (e.g., instructions, programs, etc.). In addition, the processor 210 performs various operations related to the present disclosure, such as calculation, handling, data creation, and processing. In addition, the processor 210 load data or the like from the memory 220 or stores data or the like in the memory 220. In one embodiment, the processor 210 acquires the test information 132 from the test apparatus 130 by using the communication circuit to be described later. The processor 210 determines the aforementioned first information, second information, third information, and/or fourth information on the basis of the test information 132. The processor 210 determines the gut microbiome index of the subject 110 on the basis of the first information, second information, third information, and/or fourth information. The processor 210 transmits the information 136 indicating the gut microbiome index to the device 140 of the subject by using a communication circuit.

The memory 220 stores various types of data. The data stored in the memory 220 is data that are acquired, processed or used by at least one component of the apparatus 100, and includes software (e.g., an instruction, a program, etc.). The memory 220 includes a volatile and/or nonvolatile memory. In the present disclosure, instructions or programs are software stored in the memory 220, and include an operating system for controlling the resources of the apparatus 100, an application, and/or middleware that provides various functions to the application so that the application can utilize the resources of the apparatus 100. In one embodiment, the memory 220 stores instructions that cause the processor 210 to perform an operation when the instructions are executed by the processor 210. In embodiment, the memory 220 stores information 134 about the aforementioned reference sample and/or information specifying harmful microbes and beneficial microbes, respectively. In one embodiment, the processor 210 acquires information from a predetermined server by controlling the communication circuit 230. The information obtained from the server is stored in the memory 220. In one embodiment, the information 134 about the reference sample and/or information specifying harmful microbes and beneficial microbes, respectively, are obtained from the server and stored in the memory 220.

In one embodiment, the apparatus 100 further includes a communication circuit 230. The communication circuit 230 is omitted from the apparatus 100 according to the embodiment. The communication circuit 230 performs wireless or wired communication between the apparatus 100 and the server or between the apparatus 100 and a different apparatus. For example, the communication circuit 230 performs wireless communication according to the method, such as enhanced Mobile Broadband (eMBB), Ultra Reliable Low-Latency Communication (URLLC), Massive Machine-Type Communication (MMTC), Long-Term Evolution (LTE), LTE Advance (LTE-A), New Radio (NR), Universal Mobile Telecommunications System (UMTS), Global System for Mobile communications (GSM), Code Division Multiple Access (CDMA), Wideband CDMA (WCDMA), Wireless Broadband (WiBro), Wireless Fidelity (Wi-Fi), Bluetooth, Near Field Communication (NFC), Global Positioning System (GPS), or Global Navigation Satellite System (GNSS). For example, the communication circuit 230 performs wired communication according to the method, such as Universal Serial Bus (USB), High Definition Multimedia Interface (HDMI), Recommended Standard-232 (RS-232), or Plain Old Telephone Service (POTS). In one embodiment, the communication circuit 230 performs communication with the test apparatus 130 and/or the device 140 of the subject. In one embodiment, the apparatus 100 is implemented by integration with another apparatus (e.g., the test apparatus 130). In such a case, the communication circuit 230 functions as a connection circuit or interface that connects the apparatus 100 and the other corresponding device.

In one embodiment, the apparatus 100 further includes an input/output interface 240. The input/output interface 240 is omitted from the device 100 according to the embodiment. The input/output interface 240 receives an input from a user of the apparatus 100 and output (express) information to the user. The user is an operator of the apparatus 100 belonging to the service provider 120. In one embodiment, the input/output interface 240 includes an input device and/or an output device. The input device receives information to be transmitted to at least one component of the apparatus 100 from the outside. For example, the input device includes a mouse, keyboard, touch pad, and the like. The output device provides various information of the apparatus 100 to the user in an audio-visual form. For example, the output device includes a display, a projector, a hologram, and the like. In one embodiment, the input/output interface 240 receives the information 134 about the reference sample and/or information specifying harmful microbes and beneficial microbes, respectively. In one embodiment, the input/output interface 240 display information 136 indicating the gut microbiome index to the user.

In one embodiment, the apparatus 100 includes various types of apparatuses. For example, the apparatus 100 is a computer device, a back-end server, a front-end server, a portable communication device, a portable multimedia device, or a device according to a combination of the foregoing devices. However, the apparatus 100 of the present disclosure is not limited to the aforementioned devices.

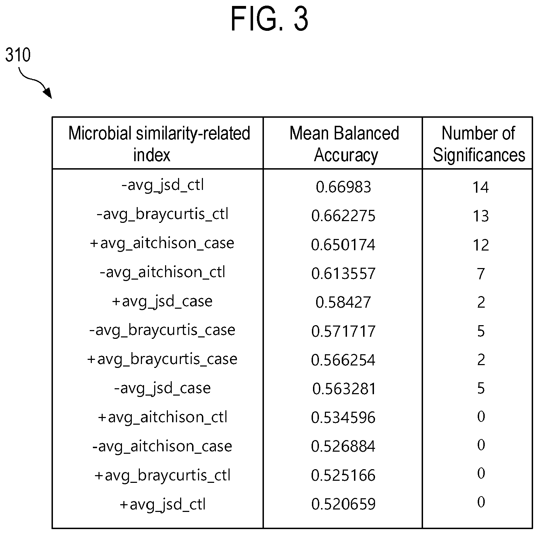

FIG. 3 is a diagram showing microbe similarity-related indexes according to one embodiment of the present disclosure. As described above, various factors can influence the ecological balance/imbalance of the gut microbiome. The factors that influence the gut ecological balance/imbalance include gut microbial similarity, proportions of harmful gut microbes, proportions of beneficial gut microbes, and/or gut microbial diversity.

**[Gut microbial similarity]**

The gut microbial similarity is described. The gut microbiome is influenced by various environmental stimuli (e.g., disease, diet, medication, lifestyle, etc.). As a result, the composition of the gut microbiome is changed. However, the gut microbiome of a healthy person has homeostasis, and thus the microbiome composition changed by the influence of the stimuli can be restored to a state before being influenced by the stimuli. The compositions of the gut microbiome of healthy persons are similar to each other due to homeostasis. However, the gut microbiome of diseased persons is in an imbalanced state and is different from the composition of the gut microbiome of healthy persons. Therefore, it can be assessed or determined whether a specific gut microbiome is in a balanced/unbalanced state, by determining the microbial similarity with the gut microbiome of healthy persons. That is, as described above, the gut microbial similarity is expressed as the degree at which the gut microbiome of the subject 110 is similar to the gut microbiome of healthy persons.

The apparatus 100 determines the gut microbial similarity by various methods. The aforementioned first information exhibits the gut microbial similarity. In one embodiment, the processor 210 of the apparatus 100 determines the gut microbial similarity on the basis of the test information 132 and the information 134 about the reference sample. The processor 210 determines the distribution of the proportion at which each microbial species is present (that is, presence proportion) in the bio-sample 112 of the subject 110 on the basis of the test information 132. The processor 210 determines a distribution of the presence proportion of each microbial species in the reference bio-sample 114 on the basis of the test information 134. In one embodiment, the distribution of the presence proportion of each microbial species in the reference sample 114 is previously calculated and included in the information 134 about the reference sample. The processor 210 determines the similarity between the distribution of the presence proportion of each microbial species in the bio-sample 112 and the distribution of the presence proportion of each microbial species in the reference sample 114. The processor 210 determines the aforementioned first information on the basis of the determined similarity.

In one embodiment, the information 134 about the reference sample is constructed, obtained, or processed from a gut microbiome database of the service provider 120 and/or a third party, and is information about an individual classified as a healthy person in each database. For example, healthy persons are a set of individuals excluding an individual corresponding to at least one of: having a disease such as irritable bowel syndrome; having an infectious disease; a BMI of a predetermined value (e.g., 25) or larger; and having taken any medication, such as a pain reliever, within the last 2 weeks.

The determination of the first information by the processor 210 is performed by calculating any one of the microbial similarity-related indexes 310. That is, the processor 210 calculates any one of the microbial similarity-related indexes 310 and determine a first information indicating the gut microbial similarity on the basis of the value. For example, for the determination of the first information, the processor 210 calculates the index +avg_jsd_ctl among the microbial similarity-related indexes 310. The index +avg_jsd_ctl represents the average of Jensen-Shannon distances between the bio-sample 112 and each of samples of healthy persons. The Jensen-Shannon distance is calculated as in Equation 1 below.

[Equation 1]

$$KL\text{-}Divergence = D_{KL}(P||Q) = \sum_i p_i \log p_i / q_i$$

$$Jensen\text{-}Shannon\ Divergence = JSD(P, Q) = \frac{1}{2}D(P||M) + \frac{1}{2}D(Q||M). \quad M = \frac{1}{2}(P + Q)$$

$$Jensen\text{-}Shannon\ Distance = \sqrt{JSD(P, Q)}$$

P and Q each represents the bio-sample 112 and a comparative sample. pi and qi represent the presence proportions of specific microbe i in the P and Q samples, respectively. M represents an average sample of P and Q and have the average of presence proportions of each microbial species in P and Q as the presence proportion of the corresponding microbial species. On the basis of the information about the bio-sample 112 and the comparative sample, the KL-divergence, Jensen-Shannon divergence, and Jensen-Shannon distance are sequentially calculated. In the same manner, the Jensen-Shannon distances be calculated for a plurality of comparative samples, and on the basis of the distances, the index +avg_jsd_ctl is determined.

The microbial similarity-related indexes 310 includes the indexes ±avg_jsd_case, ±avgj sd_ctl, ±avg_braycurtis_case, ±avg_braycurtis_ctl, ±avg_aitchison_case, ±avg_aitchison_ctl, and the like. As described above, the processor 210 determines the first information by calculating one among the indexes. The index ±avgjsd_case represents the average of Jensen-Shannon distances between the bio-sample 112 and each of samples of diseased persons. The index +avg_jsd_ctl has been described above. The index -avg_jsd_ctl has a value opposite in sign to the value of the index +avg_jsd_ctl described above. The index ±avg_braycurtis_case represents the average of Bray-Curtis dissimilarity between the bio-sample 112 and each of samples of diseased persons. The index ±avg_braycurtis_ctl represents the average of Bray-Curtis dissimilarity between the bio-sample 112 and each of samples of healthy persons. The index ±avg_aitchison_case represents the average of Aitchison distances between the bio-sample 112 and each of samples of diseased persons. The index ±avg_aitchison_ctl represents the average of Aitchison distances between the bio-sample 112 and each of samples of healthy persons. In the present disclosure, when the value of one index and the corresponding index are a positive (+) correlation in terms of the degree of accurately distinguishing a healthy person and a diseased person, the corresponding index is marked as having a "+" sign. However, when there is a negative (-) correlation, the corresponding index is marked as having a "-" sign.

The aforementioned microbial similarity-related indexes 310 are not limited to those suggested in the present disclosure, and any index that can indicate the similarity between microbial species between two samples can be used as a microbial similarity-related index 310 according to the present disclosure.

**[Indexes for determining respective information]**

In determining the first to fourth information, the type of index to be used is determined in advance. That is, a predetermined index selection process is preemptively performed to select an index to be used to determine each information, and the apparatus 100 determines the first to fourth information for the subject 110 on the basis of the preselected index. The corresponding index selection process is performed by the apparatus 100 or is performed by another electronic apparatus. On the other hand, according to the embodiment, the apparatus 100 performs index selection processes for each subject and determine the first to fourth information according to the selected indexes. In one embodiment, the gut microbiome database of the service provider 120 and/or a third party are used in the selection of indexes to be used to determine the first to fourth information, but is not limited thereto.

Hereinafter, a method of selecting the indexes to be used to determine the first to the fourth information among various indexes. The indexes to be used to determine the first, second, third, and fourth information are selected from various microbial similarity-related indexes 310, harmful microbe-related indexes 410, beneficial microbe-related indexes 510, and microbial diversity-related indexes 610 by corresponding selection methods.

First, it is determined how accurately each index distinguishes between a healthy person and a diseased person. To achieve this, values of each index are calculated for a plurality of different sample groups. The samples within one sample group (e.g., a bundle of samples in a OO test conducted by a OO hospital) are bio-samples of healthy or diseased persons, wherein the diseased persons have diseases, such as Crohn's disease, irritable bowel syndrome, and obesity.

It is determined whether each sample in a predetermined sample group is a sample of a healthy person or a sample of a diseased person, according to the value of the corresponding index. The accuracy of the corresponding index is determined according to the degree to which the corresponding index accurately distinguishes a sample of a healthy person or a diseased person as a sample of a healthy person or a diseased person, respectively.

To express the accuracy of each index by using a numerical value, the mean balanced accuracy and the number of significances for each index are determined. First, the balanced accuracy of any one index for a predetermined sample group be calculated. The balanced accuracy is calculated as in Equation 2 below.

[Equation 2]

$$Sensitivity = \frac{TP}{TP + FN}$$

$$Specificity = \frac{TN}{FP + TN}$$

$$Balanced\ accuracy = \frac{Sensitivity + Specificity}{2}$$

TP (true positive) represents the number of cases in which a sample of an actual healthy person is determined as a sample of a healthy person when the corresponding index distinguishes samples in the corresponding sample group. TN (true negative) represents the number of cases in which a sample of an actual diseased person in the corresponding sample group is determined as a sample of a diseased person. FP (false positive) represents the number of cases in which a sample of an actual diseased person in the corresponding sample group is determined as a sample of a healthy person. FN (false negative) represents the number of cases in which a sample of an actual healthy person in the corresponding sample group is determined as a sample of a diseased person. The balanced accuracy of one index for one sample group be calculated by Equation 2. In the same manner, the balanced accuracy of the corresponding index for each of the plurality of sample groups is calculated. The mean value of these balanced accuracies is calculated to derive the aforementioned mean balanced accuracy. The aforementioned number of significance refers to the number of sample groups, among a plurality of sample groups, in which healthy persons and diseased persons are significantly distinguished by the corresponding index.

In one embodiment, the mean balanced accuracy, the number of significance, and/or various other various factors are considered in the process of selecting indexes to be used to determine the first to fourth information. In one embodiment, an index having a high mean balanced accuracy is selected or an index having a large number of significances are selected. In one embodiment, a predetermined number (e.g., 10) of indexes having a high mean balanced accuracy are first selected, and an index having the largest number of significances among the selected indexes is selected as an index to be used to determine each information. On the contrary, indexes are first selected on the basis of the number of significances, and then an index having the highest mean balanced accuracy among the selected indexes is selected as an index to be used to determine each information. The mean balanced accuracy and the number of significances shown in the examples 310 have exemplary values.

In one embodiment, the correlation between indexes is further considered in the process of selecting indexes to be used to determine the first to fourth information. The correlation between indexes is calculated on the basis of, for example, Spearman correlation. The correlation between indexes (p) is calculated by Equation 3 below.

[Equation 3]

$$p = \frac{\sum_i (x_i - \bar{x})(y_i - \bar{y})}{\sqrt{\sum_i (x_i - \bar{x})^2} \sqrt{\sum_i (y_i - \bar{y})^2}}$$

In a predetermined sample group, $x_i$ is the value of index X for the i-th sample. For example, $x_3$ is the value of the +Shannon index for the third sample. $\bar{x}$ represents the average value of respective $x_i$ values. Similarly, yi represents the value of index Y for the i-th sample. $\overline{y}$ represents the average value of respective yi values.

As described above, indexes to be used to determine the first to the fourth information are first selected on the basis of the mean balanced accuracy, the number of significances, and/or other various factors. Thereafter, the correlation is calculated between the first selected indexes. A high correlation between two predetermined indexes means that the correlation between the two indexes is strong to result in a significant influence on the value of each other. On the contrary, a low correlation between two indexes means that the correlation between the two indexes is week or little to result in no significant influence on the value of each other. If the index values influence each other due to a high correlation, the corresponding index (e.g., +Shannon) not accurately indicates a specific category (e.g., gut microbial diversity). Therefore, an index having a high correlation (e.g., 0.7 or higher) compared with other indexes is excluded among the first selected indexes.

For example, the indexes -avg_jsd_ctl, -dysbiosis_index, +ben_sp_numotu, and +Shannon are first selected as indexes to be used to determine the first to fourth information. When the gut microbial similarity, the proportions of harmful gut microbes, the proportions of beneficial gut microbes, and the gut microbial diversity are each considered as one category, the corresponding indexes have the highest mean balanced accuracy in each category. If the index +shannon of the gut microbial diversity category shows a high correlation (e.g., 0.81) with the index +ben_sp_numotu of the beneficial gut microbial category, the index +shannon is excluded and then the index +numotu, which has the highest mean balanced accuracy next to the index +shannon in the gut microbial diversity category, is selected. If the index +numotu also shows a high correlation (e.g., 0.93) with the index +ben_sp_numotu, the index +numotu is excluded and then the index +invsimpson, which has the highest mean balanced accuracy next to the index +numotu in the gut microbial diversity category, is selected. If the index +invsimpson does not show a high correlation with the indexes of the other categories, the indexes -avg_jsd_ctl, - dysbiosis_index, +ben_sp_numotu, and +invsimpson are finally selected as indexes to be used to determine the first to fourth information.

**[Proportions of harmful gut microbes]**

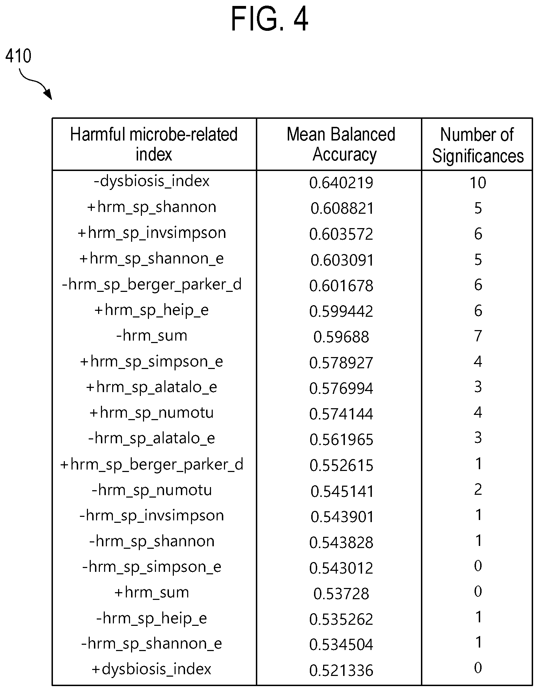

FIG. 4 is a diagram showing harmful microbe-related indexes according to one embodiment of the present disclosure. Harmful gut microbes cause gut infections and inflammatory responses or cause various diseases, such as colon cancer. Therefore, an increase in harmful gut microbes result in an imbalance in the gut microbiome. However, a sufficient quantity of microbes that live in the gut and are commensal with the human body (hereinafter, "commensal microbes") can suppress the activity or proliferation of harmful microbes through the immune modulation by commensal microbes or the like. Therefore, the proportions of harmful gut microbes are selected as one of the factors for diagnosing the gut microbiome. The proportion of harmful gut microbes is a ratio between gut commensal microbes and harmful microbes. In one embodiment of the present disclosure, already known information about harmful microbes or commensal microbes [including information (e.g., a biomarker) capable of detecting corresponding microbes] is utilized, and the information that can be determined by a person skilled in the art as harmful microbes or commensal microbes from known information is included. A statistical analysis method, such as LEfSe, or a biological analysis method, such as literature search, is used when such harmful microbes or commensal microbes are selected, but is not limited thereto.

The apparatus 100 determines the proportions of harmful gut microbes by various methods. The aforementioned second information is information indicating the proportions of harmful gut microbes. In one embodiment, the processor 210 of the apparatus 100 determines the proportions of harmful gut microbes on the basis of the test information 132. Specifically, the processor 210 determines the presence proportions of commensal microbial species in the bio-sample 112. In one embodiment, the memory 220 stores information specifying which microbes in the microbes are predetermined commensal microbe, and the processor 210 determines the presence proportions of commensal microbial species in the bio-sample 112 on the basis of the corresponding information. Meanwhile, the processor 210 determines the presence proportions of harmful microbial species in the bio-sample 112. The processor 210 uses information stored in the memory and specifying predetermined harmful microbes. The processor 210 determines the presence proportions of harmful microbial species relative to the commensal microbial species and the harmful microbial species in the bio-sample 112. That is, the corresponding proportion is the ratio of the presence proportions of harmful microbial species to the sum of the presence proportions of commensal microbial species and the presence proportions of harmful microbial species. The processor 210 determines the aforementioned second information on the basis of the presence proportions of harmful microbial species relative to commensal microbial species and harmful microbial species.

The determination process of the second information by the processor 210 is performed by calculating any one of harmful microbial proportion-related indexes 410. That is, the processor 210 calculates any one of the harmful microbial proportion-related indexes 410 and, on the basis of the value, determine the second information indicating the proportions of harmful gut microbes. For example, for determining the second information, the processor 210 calculates the index +dysbiosis_index among the harmful microbial proportion-related indexes 410. The index +dysbiosis_index represents the dysbiosis index of the bio-sample 112. The dysbiosis index is calculated as in Equation 4 below.

[Equation 4]

$$Dysbiosis\ Index\ = \frac{\sum_{k \in Pathobiont} p_k}{\sum_{k \in Pathobiont} p_k + \sum_{k \in Commensal} p_k}$$

$p_k$ represents the presence proportion of microbe k in the corresponding bio-sample 112. The "Pathobiont" represents a harmful microbe, and microbe k pertaining thereto be, for example, at least one of the microbes belonging to family *Bacteroidaceae, Desulfovibrionaceae, Oscillospiraceae, Odoribacteraceae, Lachnospiraceae, Erysipelotrichaceae, Enterococcaceae, Lactobacillaceae, Veillonellaceae, Christensenellaceae, Enterobacteriaceae, Pasteurellaceae, Fusobacteriaceae, Neisseriaceae, Veillonellaceae, Gemellaceae,* or *Porphyromonadaceae,* but is not limited thereto. The "Commensal" represents a commensal microbe, and microbe k pertaining thereto be, for example, at least one of: microbes belonging to the family *Lachnospiraceae, Eggerthellaceae, Oscillospiraceae, Rikenellaceae, Erysipelotrichaceae, Christensenellaceae, Coriobacteriaceae, Peptostreptococcaceae, Bacteroidales, Clostridiales, Erysipelotrichaceae, Clostridiales,* or *Bifidobacteriaceae*; or microbes belonging to the class *Mollicutes,* but is not limited thereto. In one embodiment, a person skilled in the art analyzes harmful microbes or commensal microbes from a microbiome database and derive the second information by using harmful microbial proportion-related indexes 410. In one embodiment, the harmful microbes include the microbes recited on Table 1, and the commensal microbes include the microbes recited on Table 2. On the basis of the information about the bio-sample 112, the dysbiosis index, that is, the index +dysbiosis_index is calculated according to Equation 4.

In Tables 1 and 2 below, the 16S rRNA sequence information corresponds to 16S rRNA sequence information of type species representing the recited microbial species.

TABLE 1

| Microbial species | 16S rRNA SEQ ID NO |
|---|---|
| Bacteroides | 1 |
| Bilophila | 2 |
| Butyricicoccus | 3 |
| Butyricimonas | 4 |
| Clostridium_g24 (Family Lachnospiraceae) | 5 |
| Clostridium_g35 (Family Lachnospiraceae) | 6 |
| Clostridium _g6 (Family Erysipelotrichaceae) | 7 |
| Enterococcus | 8 |
| Lactobacillus | 9 |
| Megasphaera | 10 |
| PAC000740_g (Family Lachnospiraceae) | 11 |
| PAC001360_g (Family Christensenellaceae) | 12 |
| Porphyromonas | 13 |
| Ruthenibacterium | 14 |

TABLE 2

| Microbial species | 16S rRNA SEQ ID NO |
|---|---|
| Acetitomaculum | 15 |
| Adlercreutzia | 16 |
| Agathob aculum | 17 |

(continued)

| Microbial species | 16S rRNA SEQ ID NO |
|---|---|
| Alistipes | 18 |
| Anaerostipes | 19 |
| Blautia | 20 |
| CCMM_g (Family Erysipelotrichaceae) | 21 |
| Christensenella | 22 |
| Coprococcus | 23 |
| Eubacterium_g17 (Family Lachnospiraceae) | 24 |
| Eubacterium_g20 (Family Lachnospiraceae) | 25 |
| Eubacterium_g23 (Family Oscillospiraceae) | 26 |
| Eubacterium_g24 (Family Lachnospiraceae) | 27 |
| Faecalibacterium | 28 |
| Frisingicoccus | 29 |
| Lachnospira | 30 |
| Lachnospiraceae_uc (Family Lachnospiraceae) | 31 |
| LT821227_g (Family Coriobacteriaceae) | 32 |
| Oscillibacter | 33 |
| PAC000194_g (Family Lachnospiraceae) | 34 |
| PAC000195_g (Family Lachnospiraceae) | 35 |
| PAC000196_g (Family Lachnospiraceae) | 36 |
| PAC000197_g (Class Mollicutes) | 37 |
| PAC000661_g (Family Oscillospiraceae) | 38 |
| PAC000692_g (Family Lachnospiraceae) | 39 |
| PAC000748_g (Family Oscillospiraceae) | 40 |
| PAC001057_g (Class Mollicutes) | 41 |
| PAC001100_g (Family Oscillospiraceae) | 42 |
| PAC001109_g (Class Mollicutes) | 43 |
| PAC001115_g (Family Christensenellaceae) | 44 |
| PAC001137_g (Family Lachnospiraceae) | 45 |
| PAC001144_g (Family Oscillospiraceae) | 46 |
| PAC001168_g (Order Clostridiales) | 47 |
| PAC001177_g (Family Lachnospiraceae) | 48 |
| PAC001200_g (Family Lachnospiraceae) | 49 |
| PAC001201_g (Family Lachnospiraceae) | 50 |
| PAC001207_g (Family Christensenellaceae) | 51 |
| PAC001217_g (Family Christensenellaceae) | 52 |
| PAC001219_g (Family Christensenellaceae) | 53 |
| PAC001231_g (Family Lachnospiraceae) | 54 |
| PAC001236_g (Order Clostridiales) | 55 |

(continued)

| Microbial species | 16S rRNA SEQ ID NO |
|---|---|
| PAC001247_g (Family Oscillospiraceae) | 56 |
| PAC001274_g (Class Mollicutes) | 57 |
| PAC001283_g (Family Lachnospiraceae) | 58 |
| PAC001296_g (Family Lachnospiraceae) | 59 |
| PAC001398_g (Family Lachnospiraceae) | 60 |
| PAC001435_g (Family Christensenellaceae) | 61 |
| PAC001437_g (Family Christensenellaceae) | 62 |
| PAC001468_g (Family Oscillospiraceae) | 63 |
| PAC001609_g (Order Clostridiales) | 64 |
| PAC002148_g (Family Oscillospiraceae) | 65 |
| PAC002518_g (Family Lachnospiraceae) | 66 |
| Paludicola | 67 |
| Romboutsia | 68 |
| Roseburia | 69 |
| Ruminococcaceae_uc (Family Oscillospiraceae) | 70 |
| Ruminococcus | 71 |
| Ruminococcus_g2 (Family Oscillospiraceae) | 72 |
| Sporobacter | 73 |
| Subdoligranulum | 74 |

The harmful microbe proportion-related indexes 410 include ±hrm_sum, ±hrm_sp_numotu, ±hrm_sp_shannon, ±hrm_sp_invsimpson, ±hrm_sp_berger_parker_d, ±hrm_sp_shannon_e, ±hrm_sp_simpson_e, ±hrm_sp_heip_e, ±hrm_sp_alatalo_e, ±dysbiosis_index, and the like. As described above, the processor 210 determines the second information by calculating one of these. Among these indexes, the index ±hrm_sum is an index indicating the sum of the presence proportions of respective harmful microbial species. The indexes ±hrm_sp_numotu, ±hrm_sp_shannon, ±hrm_sp_invsimpson, ±hrm_sp_berger_parker_d, ±hrm_sp_shannon_e, ±hrm_sp_simpson_e, ±hrm_sp_heip_e, and ±hrm_sp_alatalo_e are indexes corresponding to the indexes ±numotu, ±shannon, ±invsimpson, ±berger_parker_d, ±shannon_e, ±simpson_e, ±heip_e, and ±alatalo_e, respectively, which are the microbial diversity-related indexes to be described later. That is, the corresponding indexes are used not only as microbial diversity-related indexes, but also as harmful microbial proportion-related indexes. The index ±dysbiosis_index is a dysbiosis index.

The harmful microbe-related index used to determine the second information is previously selected according to the aforementioned index selection method. The mean balanced accuracy and the number of significances shown in the examples 410 have exemplary values. Meanwhile, the harmful microbe proportion-related indexes 410 are not limited to those suggested in the present disclosure, and any index that can indicate the proportions of harmful gut microbes is used as a harmful microbial proportion-related index 410 according to the present disclosure.

**[Proportions of beneficial gut microbes]**

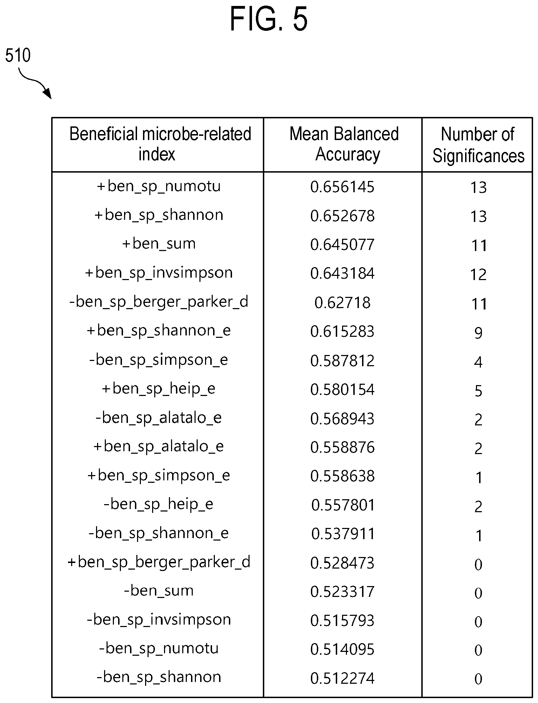

FIG. 5 is a diagram showing beneficent microbe-related indexes according to one embodiment of the present disclosure. Beneficial gut microbes can perform various functions, such as synthesizing short-chain fatty acids or inducing immune regulation and anti-inflammatory responses. Therefore, a decrease in beneficial gut microbes results in an imbalance in the gut microbiome. The proportions of beneficial gut microbes are selected as one of the factors for diagnosing the gut microbiome.

In one embodiment of the present disclosure, already known information about beneficial microbes [including information (e.g., a biomarker) capable of detecting corresponding microbes] is utilized, and the information that can be determined by a person skilled in the art as beneficial microbes from known information is included. A statistical analysis method, such as LEfSe, or a biological analysis method, such as literature search, is used when such beneficial microbes are selected, but is not limited thereto.

In one embodiment of the present disclosure, the beneficial microbes are at least one of microbes belonging to the family *Lachnospiraceae, Eggerthellaceae, Oscillospiraceae, Rikenellaceae, Erysipelotrichaceae, Christensenellaceae, Coriobacteriaceae, Peptostreptococcaceae, Bacteroidales, Clostridiales, Erysipelotrichaceae, Clostridiales,* or *Bifidobacteriaceae;* or microbes belonging to the class *Mollicutes,* but is not limited thereto. In one embodiment of the present disclosure, the beneficial microbes include the microbes recited on Table 2, but are not limited thereto.

The apparatus 100 determines the proportions of beneficial gut microbes by various methods. The aforementioned third information is information indicating the proportions of beneficial gut microbes. In one embodiment, the proportions of beneficial gut microbes are determined as the sum of the presence proportions of beneficial gut microbes. In one embodiment, the processor 210 of the apparatus 100 determines the proportions of beneficial gut microbes on the basis of the test information 132. Specifically, the processor 210 determines the presence proportions of beneficial microbial species in the bio-sample 112. The processor 210 uses information specifying predetermined beneficial microbes and stored in the memory 220. In one embodiment, the presence proportions of beneficial microbial species are presence proportions of beneficial microbial species relative to all the microbes in the gut microbiome. The processor 210 determines the aforementioned third information on the basis of the presence proportions of beneficial microbial species.

The determination process of the third information by the processor 210 is performed by calculating any one of beneficial microbial proportion-related indexes 510. That is, the processor 210 calculates any one of the beneficial microbial proportion-related indexes 510 and, on the basis of the value, determine the third information indicating the proportions of beneficial gut microbes. For example, for determining the third information, the processor 210 calculates the index +ben_sp_numotu among the beneficial microbial proportion-related indexes 510. The index +ben_sp_numotu is the number of operational taxonomic units (OTUs) of the beneficial microbial species in the bio-sample 112. The OTU is the unit in which DNA fragments obtained as a result of gene sequencing, such as NGS, are classified at a microorganism species level. That is, the number of OTUs of beneficial microbial species corresponds to the number of beneficial microbial species. The index +ben_sp_numotu is calculated as in Equation 5 below.

[Equation 5]

$$\text{Number of OTUs} = \sum_{1 \le i \le N} I_{p_i > 0}$$

N represents the number of all predetermined beneficial microbial species. $p_i$ represents the presence proportion of the i-th beneficial microbe in the bio-sample 112. Ipi represents an indicator function for OTU of the i-th beneficial microbe in the bio-sample 112. The corresponding indicator function is a function that indicates the OTU of beneficial microbial species with an presence proportion more than 0 in the bio-sample 112. On the basis of the information about the bio-sample 112, the number of OTUs of beneficial microbial species, that is, the index +ben_sp_numotu is calculated according to Equation 5.

The beneficial microbial proportion-related indexes 510 are ±ben_sum, ±ben_sp_numotu, ±ben_sp_shannon, ±ben_sp_invsimpson, ±ben_sp_berger_parker_d, ±ben_sp_shannon_e, ±ben_sp_simpson_e, ±ben_sp_heip_e, ±ben_sp_alatalo_e, and the like. As described above, the processor 210 determines the third information by calculating one of these. Among these indexes, the ±ben_sum is an index indicating the sum of the presence proportions of respective beneficial microbial species. The indexes ±ben_sp_numotu, ±ben_sp_shannon, ±ben_sp_invsimpson, ±ben_sp_berger_parker_d, ±ben_sp_shannon_e, ±ben_sp_simpson_e, ±ben_sp_heip_e, and ±ben_sp_alatalo_e are indexes corresponding to the indexes ±numotu, ±shannon, ±invsimpson, ±berger_parker_d, ±shannon_e, ±simpson_e, ±heip_e, and ±alatalo_e, respectively, which are the microbial diversity-related indexes to be described later. That is, the corresponding indexes are used not only as microbial diversity-related indexes, but also as beneficial microbial proportion-related indexes 510.

The beneficial microbe-related index used to determine the third information is previously selected according to the aforementioned index selection method. The mean balanced accuracy and the number of significances shown in the examples 510 have exemplary values. Meanwhile, the beneficial microbe proportion-related indexes 510 are not limited to those suggested in the present disclosure, and any index that can indicate the proportions of beneficial gut microbes is used as a beneficial microbial proportion-related index 510 according to the present disclosure.

**[Gut microbial diversity]**

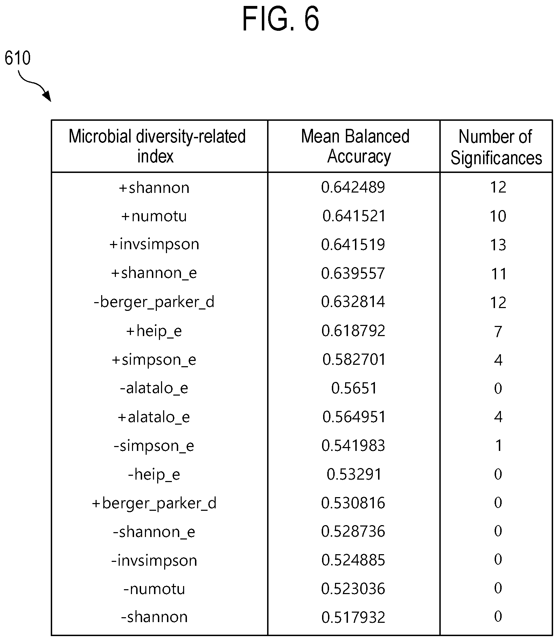

FIG. 6 is a diagram showing microbial diversity-related indexes according to one embodiment of the present disclosure. In general, one or two species of microbes have difficulty in performing all necessary functions in the gut. Each microbial species of the gut microbiome plays a role thereof. The more diverse the microbial species in the gut microbiome, the more functions in the gut can be performed, and the recovery of the functions in the gut can also be improved by various stimuli (e.g., an external disease factor). For example, diseased persons suffering from inflammatory bowel disease (IBD) and colon cancer have lower gut microbial diversity than healthy persons. Since a low gut microbial diversity results in an imbalance state of the gut microbiome, the gut microbial diversity is determined to diagnose the gut microbiome.

The apparatus 100 determines the gut microbial diversity by various methods. The aforementioned fourth information exhibits the gut microbial diversity. In one embodiment, the processor 210 of the apparatus 100 determines the gut microbial diversity on the basis of the test information 132. Specifically, the processor 210 determines the number of all the microbial species in the bio-sample 112. The processor 210 determines the degree to which the distribution of the presence proportion of each of corresponding all the microbial species in the bio-sample 112 is even. The processor 210 determines the aforementioned fourth information on the basis of the determined number of all the microbial species and evenness thereof.

The determination process of the fourth information by the processor 210 is performed by calculating any one of the microbial diversity-related indexes 610. That is, the processor 210 calculates any one of the microbial diversity-related indexes 610 and, on the basis of the value, determine the fourth information indicating the gut microbial diversity. For example, for determining the fourth information, the processor 210 calculates the index +invsimpson among the microbial diversity-related indexes 610. The index +invsimpson represents the inverse of a Simpson's index for the bio-sample 112. The index +invsimpson is calculated as in Equation 6 below.

[Equation 6]

$$Inverse\ Simpson's\ Index = \frac{1}{\sum_{1 \le i \le S} p^2_i}$$

S represents the number of all the microbial species in the bio-sample 112. $p_i$ represents the presence proportion of microbe i in the bio-sample 112. On the basis of the information about the bio-sample 112, the index +invsimpson be calculated according to Equation 6.

The microbial diversity-related indexes 610 are ±numotu, ±shannon, ±invsimpson, ±berger_parker_d, ±shannon_e, ±simpson_e, ±heip_e, ±alatalo_e, and the like. As described above, the processor 210 determines the fourth information by calculating one of the indexes. The index ±numotu represents the number of OTUs. The index ±shannon represents the Shannon's Index. The index ±invsimpson represents the inverse of the Simpson Index. The index ±berger_parker_d is Berger Parker's Index. The index ±shannon_e means Shannon's Equitability. The index ±simpson_e means Simpson's Evenness. The index ±heip_e means Heip's Index. The index ±alatalo_e means Alatalo Index. For example, when calculation is performed by inputting the information about the number of each microbial species in the biological sample 112, the presence proportion thereof, and the like into any one of the aforementioned microbial diversity-related indexes 610, the resulting value (value of the corresponding index) reflects and show how many microbial species the corresponding bio-sample 112 has.

The microbial diversity-related index used to determine the fourth information is previously selected according to the aforementioned index selection method. The mean balanced accuracy and the number of significances shown in the examples 610 have exemplary values. Meanwhile, the microbial diversity-related indexes 610 are not limited to those suggested in the present disclosure, and any index that can indicate the gut microbial diversity is used as a microbial diversity-related index 610 according to the present disclosure.

**[Gut microbiome index]**

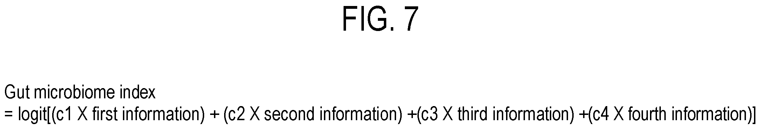

FIG. 7 is a diagram showing a function 700 for determining the gut microbiome index according to one embodiment of the present disclosure. As described above, the processor 210 determines the first, second, third, and/or fourth information about the gut microbiome of the subject 110 on the basis of the test information 132 about the bio-sample 112 of the subject 110 in FIG. 1. In one embodiment, the process 210 determines the gut microbiome index of the subject 110 on the basis of at least one information selected from the first, second, third, and fourth information.

In one embodiment, the processor 210 applies predetermined weights c1, c2, c3, and c4 to the first, second, third, and/or fourth information, respectively. In one embodiment, the weights c1, c2, c3, and c4 have different values for the first, second, third, and/or fourth information, respectively. For example, the weights c1, c2, c3, and c4 have values of -1.50751, -0.64560, 0.98436, and 0.07459, respectively. In one embodiment, the weights c1, c2, c3, and c4 are previously determined by the linear mixed effect model. Each of the first to fourth information is set as an independent variable of the linear mixed effect model, and the degree to which a healthy person and a diseased person are accurately distinguished by each information is set as a dependent variable. A change in each of the first to fourth information values has a mixed influence on a dependent variable value. On the basis of the linear mixed effect model indicating the influence, the respective weights c1, c2, c3, and c4 are determined. In one embodiment, the memory 220 stores information indicating the determined weights c1, c2, c3, and c4.

In one embodiment, the processor 210 determines the gut microbiome index on the basis of at least one selected from respective information to which weights are applied. In one embodiment the processor 210 determines the gut microbiome index on the basis of the sum of the first to fourth information to which corresponding weights are applied. In one embodiment, the processor 210 determines the gut microbiome index on the basis of the function 700 shown in the drawing. The function 700 is a function that performs a logit operation on a value obtained by adding up the first to fourth information to which corresponding weights are applied. In one embodiment, the memory 220 stores information indicating the function 700 for determining the gut microbiome index.

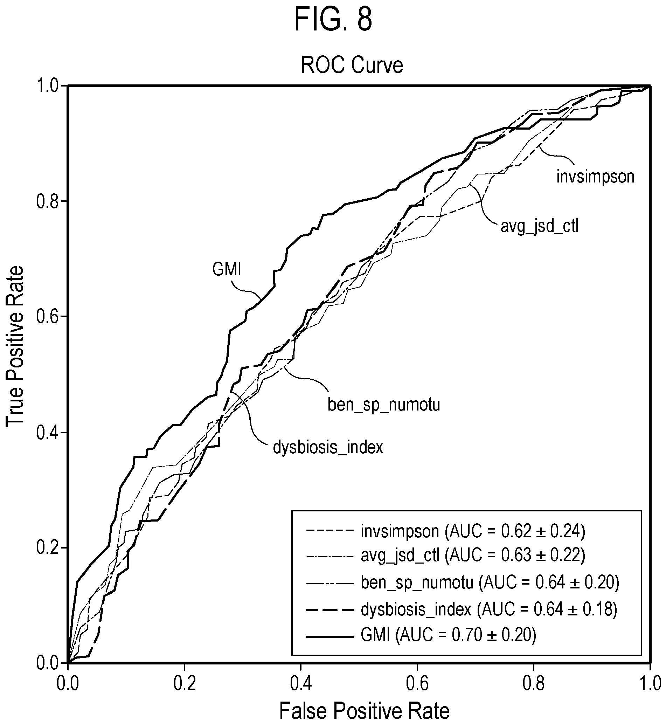

FIG. 8 is a diagram showing the test results 800 of accuracy of the gut microbiome index according to one embodiment of the present disclosure. The test results 800 show an average receiver operating characteristics (ROC) curve of the gut microbiome index and average ROC curves of other indexes according to the present disclosure. In the test results 800, the horizontal axis represents the false positive rate (FPR). FPR represents the rate at which the corresponding index incorrectly classifies a sample of an actual diseased person as a sample of a healthy person. FPR is calculated as in Equation 7 below. In the test results 800, the longitudinal axis represents the true positive rate (TPR). TPR represents the rate at which the corresponding index correctly classifies a sample of an actual healthy person as a sample of a healthy person. TPR is calculated in the same manner as the aforementioned sensitivity.

[Equation 7]

$$FPR(False\ Positive\ Rate) = \frac{FP}{TN + FP}$$

As an ROC curve is biased to the upper left, it means that the corresponding index accurately performs the classification between a healthy person and a diseased person. On ROC curves, false negatives (determining a healthy person as a diseased person) are fewer toward the left side and false positives (determining a diseased person as a healthy person) are fewer toward the upper side. An average ROC curve is the average of ROC curves for a plurality of samples.

The test results (800) confirmed that the ROC curve (gmi_lmm) of the gut microbiome index is biased to the upper left side compared with the ROC curves of the indexes +invsimpson, +avgjsd_ctl, +ben_sp_numotu, and +dysbiosis_index. That is, it can be confirmed that the accuracy of the gut microbiome index is higher than those of the indexes +invsimpson, +avg_jsd_ctl, +ben_sp_numotu, +dysbiosis_index.

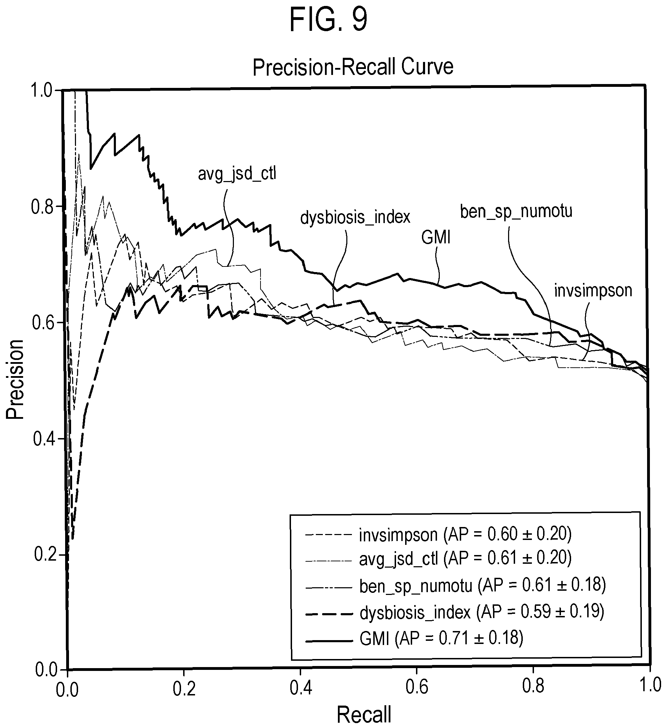

FIG. 9 is a diagram showing the test results 900 of accuracy of the gut microbiome index according to one embodiment of the present disclosure. The test results 900 show a precision-recall curve of the gut microbiome index and precision-recall curves of other indexes according to the present disclosure. In the test results 900, the horizontal axis represents recall (true positive rate). The recall represents the rate at which the corresponding index correctly classifies a sample of an actual healthy person as a sample of a healthy person. The recall is calculated in the same manner as the aforementioned sensitivity. In the test results 900, the longitudinal axis represents precision (positive predictive value). The precision represents the rate of samples of actual healthy persons among the samples classified by the corresponding index as being a sample of a healthy person. The precision is calculated as in Equation 8 below.

[Equation 8]

$$Precision = \frac{TP}{TP + FP}$$

As the precision-recall curve is biased to the upper right, it means that the corresponding index accurately performs the classification between a healthy person and a diseased person. On precision-recall curves, the recall is higher toward the right side and the precision is higher toward the upper side. In general, when one curve includes the other curves on the basis of the upper right, the including corresponding curve have excellent performance compared with the other curves.

The test results (900) confirmed that the precision-recall curve (gmi_lmm) of the gut microbiome index is biased to the upper right side compared with the precision-recall curves of the indexes +invsimpson, +avgjsd_ctl, +ben_sp_numotu, and +dysbiosis_index. That is, it can be confirmed that the accuracy of the gut microbiome index is higher than those of the indexes +invsimpson, +avg_jsd_ctl, +ben_sp_numotu, +dysbiosis_index.

**[Determining enterotype]**

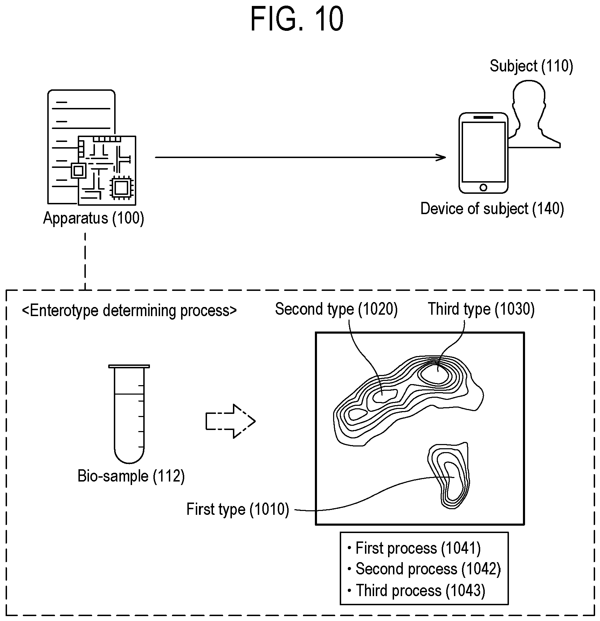

FIG. 10 is a diagram showing an operation of an apparatus 100 according to one embodiment of the present disclosure. The apparatus 100 according to one embodiment of the present disclosure determines the enterotype of the subject by comprehensively considering various factors. As described above, the processor 210 of the apparatus 100 acquires the test information 132 about the bio-sample 112 of the subject 110 from the test apparatus 130. The processor 210 determines the enterotype of the subject 110 on the basis of the test information 132.

In one embodiment, the processor 210 classifies the enterotype as a first type 1010, a second type 1020, and/or a third type 1030. The first type 1010 is a type in which inflammation-inducing microbes are abundant in the gut microbiome. The first type 1010 is an enterotype of having similar microbiomes to the gut microbiome of a diseased person. The second type 1020 is a type in which the genus *Prevotella* microbes are abundant in the gut microbiome. The second type 1020 has a similar gut microbiome to a foraging person. The third type 1030 is a type in which the genus *Bacteroides* microbes are abundant in the gut microbiome. The third type 1030 has a similar gut microbiome to Western persons.

The processor 210 determines the enterotype of the subject 110 by various methods. According to embodiments, the enterotype of the subject 110 is determined from the bio-sample 112 through a single process, or the enterotype of the subject 110 is determined by sequentially performing two or more processes. In one embodiment, the processor 210 determines the enterotype of the subject 110 on the basis of at least one selected from a first process 1041, a second process 1042, and a third process 1043. When two or more processes are performed, the processor 210 sequentially/parallelly performs the two or more processes according to a predetermined order.

The first process 1041 will be first described. In the first process 1041, the processor 210 determines the bio-sample 112 as a sample corresponding to one of the first type 1010, the second type 1020, and the third type 1030, on the basis of the Jensen-Shannon distance. The memory 220 store information about a reference sample corresponding to each of the first type 1010, the second type 1020, and/or the third type 1030. The processor 210 determines the Jensen-Shannon distance between the bio-sample 112 and the reference sample of each enterotype. The processor 210 determines, on the basis of the Jensen-Shannon distance, which type of reference sample the bio-sample 112 is similar to, and then determine the enterotype corresponding to the bio-sample 112 according to the determined type. For example, if the Jensen-Shannon distance between the bio-sample 112 and the reference sample corresponding to the first type 1010 is close, the processor 210 determines that the corresponding bio-sample 112 corresponds to the first type 1010. In one embodiment, the similarity that the corresponding bio-sample needs to have with the reference sample related to a predetermined enterotype in order to be classified as the corresponding enterotype is made on the basis of an arbitrary clustering algorithm (e.g., R Package).

Next, the second process 1042 will be described. In the second process 1042, the processor 210 determines the bio-sample 112 as a sample corresponding to one of the second type 1020 and the third type 1030, on the basis of the genus *Prevotella* and/or *Bacteroides* microbes. In one embodiment, the processor 210 performs the second process 1042 on the basis of the test information 132. In one embodiment, the test information 132 includes information indicating the presence proportions of the genus *Prevotella* and/or *Bacteroides* microbes in the bio-sample 112. In one embodiment, according to the determination that the presence proportions of the genus *Prevotella* microbes in the bio-sample 112 satisfies a predetermined level, the processor 210 determines the enterotype of the subject 110 as the second type 1020 or the third type 1030. In one embodiment, the processor 210 determines the enterotype as the second type 1020 when the presence proportions of the genus *Prevotella* microbes in the total gut microbes of the bio-sample 112 exceeds approximately 3%, and the enterotype as the third type 1030 if does not exceed approximately 3%. In one embodiment, according to the determination that the Prevotella-to-Bacteroides ratio in the bio-sample 112 satisfies a predetermined standard, the processor 210 determines the enterotype of the subject 110 as the second type 1020 or the third type 1030. In one embodiment, the processor 210 determines the enterotype as the second type 1020 when the Prevotella-to-Bacteroides ratio in the bio-sample 112 exceeds 0.5, and the enterotype as the third type 1030 if does not exceed 0.5.

Next, the third process 1043 will be described. In the third process 1043, the processor 210 determines the bio-sample 112 as the first type 1010, on the basis of the level of a specific biomarker and/or the gut microbiome index. In one embodiment, the processor 210 determines the enterotype of the subject 110 on the basis of the level (measured value) of at least one biomarker relative to the bio-sample 112. In one embodiment, the test information 132 includes the level of at least one biomarker relative to the corresponding bio-sample 112. In one embodiment, at least one of corresponding biomarkers is an inflammation-specific biomarker. In one embodiment, the processor 210 determines the enterotype of the subject 110 as the first type 1010 on the basis of the level of the inflammation-specific biomarker relative to the bio-sample 112. In one embodiment, according to the determination that the level of the corresponding inflammation-specific biomarker satisfies a predetermined standard (hereinafter, "first standard"), the processor 210 determines the enterotype of the subject 110 as the first type 1010. In the present disclosure, one variable satisfying a predetermined standard means that the corresponding variable is no less than, no more than, more than, or less than the reference value of the corresponding standard according to embodiments. In one embodiment, the corresponding inflammation-specific biomarker is at least one selected from: microbes belonging to the phylum Proteobacteria; microbes belonging to the genus *Fusobacterium*; microbes belonging to the genus Prevotella; microbes belonging to the family *Bacteroidaceae, Desulfovibrionaceae, Oscillospiraceae, Odoribacteraceae, Lachnospiraceae, Erysipelotrichaceae, Enterococcaceae, Lactobacillaceae, Veillonellaceae, Christensenellaceae, Enterobacteriaceae, Pasteurellaceae, Fusobacteriaceae, Neisseriaceae, Veillonellaceae, Gemellaceae,* or *Porphyromonadaceae;* or microbes recited on Table 1, but is not limited thereto, and any microbe that is known as an inflammation-specific biomarker to a person skilled in the art is also used to determine the first type. In one embodiment, according to the determination that the presence proportions of microbes belonging to the phylum Proteobacteria among all the gut microbes in the bio-sample 112 exceeds approximately 10% or the presence proportions of microbes belonging to the genus *Fusobacterium* among all the gut microbes in the bio-sample 112 exceeds approximately 1%, the processor 210 determines the enterotype of the subject 110 as the first type 1010. In one embodiment, according to the determination that the presence proportion of microbes belonging to the phylum Proteobacteria among all the gut microbes in the bio-sample 112 exceeds approximately 10% and the presence proportion of microbes belonging to the genus *Fusobacterium* among all the gut microbes in the bio-sample 112 exceeds approximately 1%, the processor 210 determines the enterotype of the subject 110 as the first type 1010. In one embodiment, the level of a specific biomarker is expressed as a percentage of the presence proportion of microbes belonging to the corresponding biomarker among all the gut microbes in the bio-sample 112 of the subject 110.

As described above, the processor 210 determines the gut microbiome index of the subject 110 on the basis of the test information 132. In one embodiment, the processor 210 determines the enterotype of the subject 110 on the basis of the gut microbiome index determined for the bio-sample 112. In one embodiment, according to the determination that the gut microbiome index satisfies a predetermined standard (hereinafter, "second standard"), the processor 210 determines the enterotype of the subject 110 as the first type 1010. In one embodiment, according to the determination that the gut microbiome index is smaller than approximately 40, the processor 210 determines the enterotype of the subject 110 as the first type 1010.

In one embodiment, the processor 210 determines the enterotype of the subject 110 as the first type 1010 on the basis of a combination of the level of the inflammation-specific biomarker relative to the bio-sample 112 and the gut microbiome index of the subject 110. In one embodiment, according to the determination that the level of the inflammation-specific biomarker relative to the bio-sample 112 satisfies the aforementioned first standard or the gut microbiome index of the subject 110 satisfies the aforementioned second standard, the processor 210 determines the enterotype of the subject 110 as the first type 1010. In one embodiment, according to the determination that the level of the inflammation-specific biomarker relative to the bio-sample 112 satisfies the aforementioned first standard and the gut microbiome index of the subject 110 satisfies the aforementioned second standard, the processor 210 determines the enterotype of the subject 110 as the first type 1010.

As described above, at least two processes selected from the first process 1041, the second process 1042, and the third process 1043 are sequentially/parallelly performed according to a predetermined order. In one embodiment, the processor 210 performs the third process 1043. First, the processor 210 determines whether the enterotype of the subject 110 is the first type 1010, on the basis of one of the aforementioned methods of the third process 1043. If the enterotype of the subject 110 is determined as the first type 1010, the processor 210 determines the corresponding enterotype as the first type 1010. In one embodiment, when the enterotype of the subject 110 is not determined as the first type 1010, the processor 210 further performs, on the corresponding subject 110, the classification according to the aforementioned first process 1041 and/or the second process 1042. For example, the processor 210 determines the enterotype as the second type 1020 if the presence proportion of the genus *Prevotella* microbes in the corresponding bio-sample 112 exceeds approximately 3%, and the enterotype as the third type 1030 if does not exceed approximately 3.

In one embodiment, the processor 210 performs the second process and then perform the third process 1043. First, the processor 210 determines whether the enterotype of the subject 110 is the second type 1020 or the third type 1030, on the basis of one of the aforementioned methods of the second process 1042. Second, the processor 210 again performs the third process 1043 on the subject 110, classified as the second type 1020 or the third type 1030, to determine whether the bio-sample 112 of the subject 110 corresponds to the aforementioned conditions for being determined as the first type 1010. If the bio-sample 112 satisfies the aforementioned conditions for being determined as the first type 1010, the processor 210 again determines (overrides) the enterotype of the subject 110 as the first type 1010.

In one embodiment, the processor 210 determines the enterotype according to the order of the first process 1041, the second process 1042, and the third process 1043. First, the processor 210 determines the bio-sample 112 as one of the first type 1010, the second type 1020, the third type 1030, on the basis of the first process 1041. Then, the processor 210 re-classifies the bio-sample 112 according to the second process 1042. For example, when the bio-sample 112 has been determined as the third type 1030 in the first process 1041, the bio-sample 112 is again determined as the second type 1020 or is maintained as the third type 1030 according to the second process 1042. Thereafter, the processor 210 performs the third process 1043. In the case where the bio-sample 112 is classified as the second type 1020 or the third type 1030 through the preceding first process 1041 and the second process 1042, the processor 210 determines (overrides) the corresponding bio-sample 112 as the first type 1010 again if the bio-sample 112 satisfies the aforementioned conditions for being determined as the first type 1010.

In one embodiment, the processor 210 further sub-classifies the enterotype of the subject 110 compared with the first type 1010 to the third type 1030. In one embodiment, the gut microbiome index is utilized for sub-classification of the enterotype. In one embodiment, the position of the gut microbiome index of the subject 110 in the gut microbiome index distribution of all subjects is utilized for sub-classification of the enterotype. For example, on the basis of what upper or lower percentage of the gut microbiome indexes within the corresponding enterotype (e.g., second type 1020) the gut microbiome index of the subject 110 is positioned, the enterotype of the subject 110 is further sub-classified (e.g., second-first type, second-second type, second-third type, etc.). For example, among the subjects corresponding to the second type (1020), a subject having a gut microbiome index of less than approximately 40 is sub-classified as a second-first type; a subject having a gut microbiome index of more than approximately 40 and less than approximately 70 is sub-classified as a second-second type; and a subject having a gut microbiome index of more than approximately 70 is sub-classified as a second-third type.

In one embodiment, the aforementioned first process 1041 is additionally performed with respect to the already classified enterotype, for sub-classification of the enterotype. That is, first, when the enterotype is classified as one of the first type 1010 to the third type 1030, the classified enterotype is sub-classified through the Jensen-Shannon distance between the corresponding bio-sample and a reference sample of each sub-enterotype within the classified enterotype. The first process 1041 is as described above.

In one embodiment, a characteristic biomarker is previously selected (determined) for each sub-classified enterotype (e.g., second-first type, etc.) according to one of the aforementioned methods. The sub-enterotype of the subject 110 is determined using a previously selected corresponding biomarker. The test information 132 includes the level of each biomarker relative to the bio-sample 112 of the subject 110. The processor 210 determines the sub-enterotype of the subject 110 on the basis of the test information 132. In one embodiment, biomarkers characteristic of each sub-enterotype is previously selected on the basis of various literatures.

**[Providing customized solutions suited to enterotvpel**

When the enterotype of the subject 110 is determined by the aforementioned processes, the processor 210 acquires gut management information 1060 related to the determined enterotype from the memory 220. In one embodiment, the memory 220 stores gut management information for each enterotype. In one embodiment, the gut management information 1060 indicates a suggestion for regulating the gut microbiome of the subject 110 having the corresponding enterotype.

The processor 210 transmits the information indicating the determined enterotype and/or the gut management information 1060 related to the corresponding enterotype to the device 140 of the subject by using the communication circuit 230. In one embodiment, the processor 210 transmits the information indicating the determined enterotype, the gut management information 1060 related to the corresponding enterotype, and/or the information 136 indicating the gut microbiome index of the subject 110 to the device 140 of the subject by using the communication circuit 230.

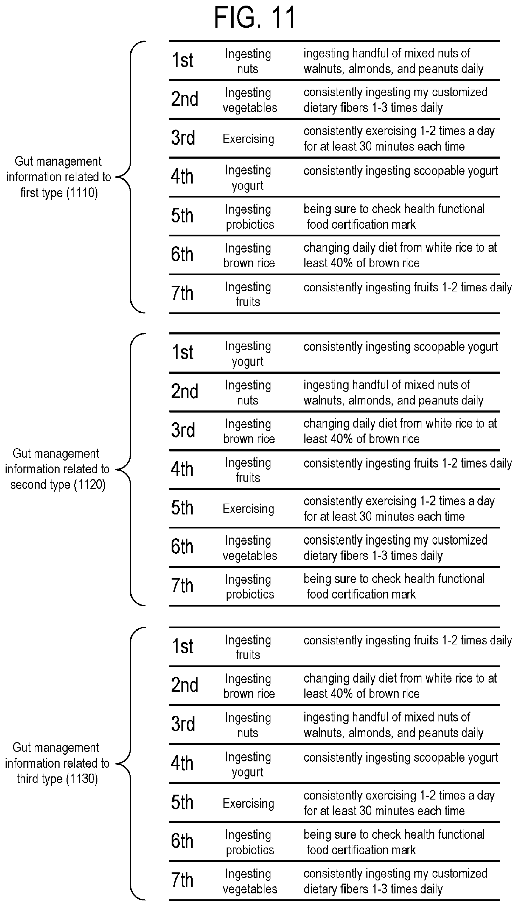

FIG. 11 is a diagram showing gut management information 1060 according to one embodiment of the present disclosure. The gut management information 1060 includes one or more suggestions for regulating the gut microbiome. The gut management information 1060 is related to a specific enterotype. In one embodiment, the enterotype means the aforementioned first, second, and third enterotypes 1010, 1020, and 1030 or means the enterotype according to the sub-classification, wherein the sub-classification is a classification according to the age group. The gut management information 1060 includes a gut microbiome regulating suggestion capable of significantly changing (improving) the gut microbiome of the subject 110 having the related enterotype. That is, the gut management information 1060 includes a gut microbiome regulating suggestion capable of: significantly changing (increasing) the gut microbiome index of the subject 110 having the corresponding enterotype; significantly changing (increasing) the gut microbiome index according to the age group of the subject 110 having the corresponding enterotype; changing (reducing) inflammation-inducing microbes living in the gut of the subject 110 having the corresponding enterotype; changing (reducing) inflammation-inhibiting microbes living in the gut of the subject 110 having the corresponding enterotype; or alleviating the constipation and/or diarrhea symptoms of the subject 110 having the corresponding enterotype.

In one embodiment, the gut microbiome regulating suggestion includes at least one selected from ingesting specific probiotics corresponding to the corresponding enterotype, ingesting specific food, and applying specific life habits. In one embodiment, the gut microbiome regulating suggestion includes information specifying one or more probiotics capable of significantly changing the gut microbiome in the gut having the corresponding enterotype, information specifying one or more foods, and/or information specifying one or more life habits. In one embodiment, the probiotics is a customized health functional food. In one embodiment, the information specifying probiotics is information suggesting a customized health functional food containing at least one species of microbes suggested to a subject according to the embodiment.

In one embodiment, the gut microbiome regulating suggestion includes information suggesting, to a subject classified as the first type 1010, probiotics containing at least one species of microbes including at least one *Lactobacillus paracasei,* at least one *Lactobacillus fermentum,* at least one *Lactobacillus plantarum* group, and/or at least one *Lactococcus lactis* group. The probiotics information suggested to the subject 1010 of the first type 1010 includes further suggesting at least one microbial species representing a subgroup among microbes belonging to the same genus in order to increase diversity. A subject ingesting the suggested probiotics increases the gut microbiome index after ingesting strains. The subjects classified as the first type are suggested to ingest: (i) *Lactobacillus paracasei* that includes *Lactobacillus paracasei subsp. paracasei, Lactobacillus paracasei subsp. tolerance,* or *Lactobacillus paracasei subsp. tolerance* HM0866 (accession number: KCTC14409BP, same as below); (ii) *Lactobacillus fermentum* that includes *Lactobacillus fermentum* HM0740 (accession number: KCTC14406BP, same as below); (iii) a *Lactobacillus plantarum* group that includes at least one of *Lactobacillus plantarum, Lactobacillus pentosus, Lactobacillus paraplantarum, Lactobacillus fabifermentans, Lactobacillus xiangfangensis, Lactobacillus herbarum, Lactobacillus modestisalitolerans,* or subsp. thereof, wherein *Lactobacillus plantarum* includes *Lactobacillus plantarum subsp. plantarum, Lactobacillus plantarum subsp. argentoratensis,* or *Lactobacillus plantarum subsp. plantarum* HM0782 (accession number: KCTC14407BP, same as below); and (iv) a *Lactococcus lactis* group that includes *Lactococcus lactis subsp. Lactis* (e.g., *Lactococcus lactis subsp. lactis* HM0850 (accession number: KCTC14408BP, same as below)), *Lactococcus lactis subsp. hordniae, Lactococcus lactis subsp. cremoris,* or *Lactococcus lactis subsp. tructae.*

In one embodiment, the gut microbiome regulating suggestion includes information suggesting, to a subject classified as the second type 1020, probiotics containing at least one species of microbes including at least one *Lactobacillus fermentum* and/or at least one *Lactococcus lactis* group. A subject ingesting the suggested probiotics increases the gut microbiome index after ingesting strains. The subjects classified as the second type are suggested to ingest: (i) *Lactobacillus fermentum* that includes *Lactobacillus fermentum* HM0740; and (ii) a *Lactococcus lactis* group that includes *Lactococcus lactis subsp. Lactis* (e.g., *Lactococcus lactis subsp. lactis* HM0850), *Lactococcus lactis subsp. hordniae, Lactococcus lactis subsp. cremoris,* or *Lactococcus lactis subsp. tructae.*

In one embodiment, the gut microbiome regulating suggestion includes information suggesting, to a subject classified as the third type 1030, probiotics containing at least one species of microbes including at least one *Lactobacillus fermentum,* at least one *Lactobacillus plantarum* group, and/or at least one *Lactococcus lactis* group. The probiotics information suggested to the subject having third type 1030 includes further suggesting a microbial species with increased activity to use dietary fibers in order to enhance such ability. A subject ingesting the suggested probiotics increases the gut microbiome index after ingesting strains. The subjects classified as the third type are suggested to ingest: (i) *Lactobacillus fermentum* that includes *Lactobacillus fermentum* HM0740; (ii) a *Lactobacillus plantarum* group that includes at least one of *Lactobacillus plantarum, Lactobacillus pentosus, Lactobacillus paraplantarum, Lactobacillus fabifermentans, Lactobacillus xiangfangensis, Lactobacillus herbarum, Lactobacillus modestisalitolerans,* or subsp. thereof, wherein *Lactobacillus plantarum* includes *Lactobacillus plantarum subsp. plantarum, Lactobacillus plantarum subsp. argentoratensis,* or *Lactobacillus plantarum subsp. plantarum* HM0782; and (iii) a *Lactococcus lactis* group that includes *Lactococcus lactis subsp. Lactis* (e.g., *Lactococcus lactis subsp. lactis* HM0850), *Lactococcus lactis subsp. hordniae, Lactococcus lactis subsp. cremoris,* or *Lactococcus lactis subsp. tructae.*

In one embodiment, one or more gut microbiome regulating suggestions specified by the gut management information 1060 be prioritized. The corresponding prioritization is differently performed according to the enterotype related to the gut management information 1060. That is, one or more gut microbiome regulating suggestions in the gut management information 1060 have priority according to the degree at which each suggestion changes (increases) the gut microbiome index of the subject 110 with the corresponding enterotype.

In the shown example 1110 of the gut management information for the first type, the gut management information 1060 indicates gut microbiome regulating suggestions, such as "ingesting nuts", "ingesting vegetables", "exercising", "ingesting yogurt", "ingesting probiotics", "ingesting brown rice", and "ingesting fruits". The corresponding gut microbiome regulating suggestions are prioritized from first to seventh. As for the suggestion "ingesting probiotics", the gut management information 1060 further includes information specifying which probiotics to ingest. In a similar manner as described above, the example 1120 of the gut management information related to the second type and the example 1130 of the gut management information of the gut management information related to the third type also includes information about gut microbiome regulating suggestions for the subjects 110 of the second type 1020 and the third type 1030.

In one embodiment, the gut management information 1060 includes different gut microbiome regulating suggestions according to at least one of the factors, such as an enterotype, a level of the gut microbiome, a level of a specific biomarker, a level of Prevotella, and a level of Bacteroides. In one embodiment, the gut management information 1060 differently prioritizes the gut regulating suggestions according to at least one of the factors, such as an enterotype, a level of the gut microbiome, a level of a specific biomarker, a level of Prevotella, and a level of Bacteroides.

In one embodiment, the gut management information 1060 includes the classification information 1070 of the subject 110 according to the gut microbiome index of the subject 110. Th subject 110 is classified according to the gut microbiome index thereof. The classification information 1070 is information indicating which classification the subject 110 belongs to, on the basis of the gut microbiome index. In one embodiment, the gut microbiome regulating suggestions of the gut management information 1060 is related to a specific classification of the subject 110 indicated by the classification information 1070. That is, the gut management information 1060 includes a gut microbiome regulating suggestion capable of significantly changing (improving) the gut microbiome of the subject 110 classified as a specific type.

In one embodiment, the subjects 110 are classified in various manners on the basis of the gut microbiome index. In one embodiment, the higher the gut microbiome index, the subject is classified as a healthy person, and the lower the gut microbiome index, the subject is classified as a diseased person. In one embodiment, one or more sections are assigned to the gut microbiome index, and the subject 110 is classified according to the sections. For example, when the gut microbiome index is expressed as a value between 0 and 100, the subject 110 is classified as "dangerous" if the gut microbiome index is not smaller than 0 and smaller than 40, as "cautious" if not smaller than 40 and smaller than 70, and as "good" if not smaller than 70 and not greater than 100. Depending on the embodiment, the number of sections assigned to the gut microbiome index or the range of sections is set differently.

In one embodiment, the sections assigned to the gut microbiome index be set on the basis of a distribution of gut microbiome indexes of all subjects. For example, the distribution of gut microbiome indexes of all subjects is divided into quartiles. In such a situation, the subject 110 is classified as "balanced" if the gut microbiome index is less than the top 25%, as "slightly balanced" if not less than top 25% and less than top 50%, as "slightly imbalanced" if not less than top 50% and less than top 75%, and as "imbalanced" if not less than 75% and not more than 100%.

In one embodiment, the sections assigned to the gut microbiome index are set on the basis of the accuracy of the gut microbiome index. The accuracy of the gut microbiome index is calculated by obtaining the gut microbiome index for each sample of one or more sample groups and reviewing the results. For example, it is assumed that a sample with a gut microbiome index of greater than a predetermined value (e.g., 47) was highly likely to be a sample of an actual healthy person and a sample with a gut microbiome index of smaller than the corresponding value was highly likely to be a sample of an actual diseased person. In this case, the sections of the gut microbiome index are set according to the corresponding reference value (e.g., 47). That is, the subject 110 is classified as "imbalanced" if the gut microbiome index is smaller than a reference value (e.g., 47) and as "balanced" if not smaller than the reference value (e.g., 47).

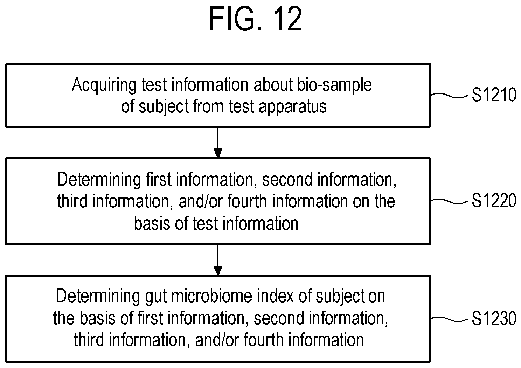

FIG. 12 is a diagram showing a gut microbiome index determining method 1200 according to one embodiment of the present disclosure. A gut microbiome index determining method 1200 according to various embodiments of the present disclosure is performed by the apparatus 100. The present method 1200 includes: acquiring test information about a bio-sample of a subject from a test apparatus (S1210); determining first, second, third, and/or fourth information on the basis of the test information (S1220); and/or determining the gut microbiome index of the subject on the basis of the first, second, third, and/or fourth information (S1230).

In step S1210, the processor 210 acquires the test information 132 on the bio-sample 112 of the subject 110 from the test apparatus 130 by using the communication circuit 230. In step S1220, the processor 210 determines the first, second, third, and/or fourth information on the basis of the test information 132. In step S1230, the processor 210 determines the gut microbiome index of the subject 110 on the basis of the first, second, third, and/or fourth information.

In one embodiment, the method 1200 further includes transmitting, by the processor 210, information 136 indicating the gut microbiome index to the device 140 of the subject by using the communication circuit 230.

In one embodiment, the memory 220 stores the information 134 about the reference samples. The method 1200 further includes: determining, by the processor 210, the similarity between the presence proportion of each microbial species in the bio-sample 112 and the presence proportion of each microbial species in the reference sample 114 on the basis of the test information 132 and/or the information 134 about the reference sample, and/or determining the first information on the basis of the determined similarity.

In one embodiment, the present method 1200 further includes determining, by the processor 210, the presence proportions of harmful microbial species relative to predetermined commensal microbial species and harmful microbial species in the bio-sample 112 on the basis of the test information 132, and/or determining the second information on the basis of the determined presence proportions.

In one embodiment, the present method 1200 further includes determining, by the processor 210, the presence proportions of predetermined beneficial microbial species in the bio-sample 112 on the basis of the test information 132, and/or determining the third information on the basis of the determined presence proportions.

In one embodiment, the present method 1200 further includes determining, by the processor 210, on the basis of the test information 132, the number of all the microbial species in the bio-sample 112 and the degree to which the distribution of the presence proportion of each of all the microbial species is even, and/or determining the fourth information on the basis of the determined number of all the microbial species and evenness.

In one embodiment, the present method 1200 further includes determining, by the processor 210, the gut microbiome index by applying predetermined weights $c_1$, $c_2$, $c_3$, and $c_4$ to the first, second, third, and/or fourth information, respectively.

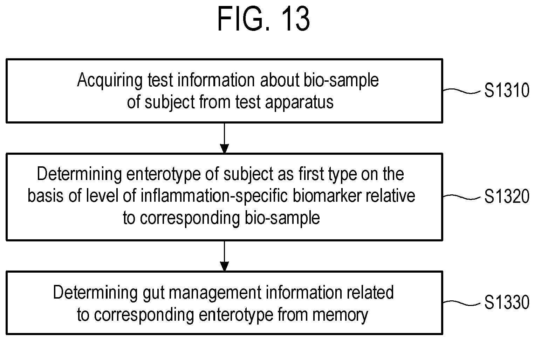

FIG. 13 is a diagram showing an enterotype determining method 1300 according to one embodiment of the present disclosure. An enterotype determining method 1300 according to various embodiments of the present disclosure is performed by the apparatus 100. The present method 1300 includes: acquiring test information about a bio-sample of a subject from a test apparatus (S13 10); determining the enterotype of a subject as a first type on the basis of the level of an inflammation-specific biomarker relative to the corresponding bio-sample (S1320); and determining gut management information related to the corresponding enterotype from memory (S1330).

In step S13 10, the processor 210 acquires the test information 132 about the bio-sample 112 of the subject 110 from the test apparatus 130 by using the communication circuit 230. In step S1320, the processor 210 determines the enterotype of the subject 110 as the first type 1010 on the basis of the level of the inflammation-specific biomarker relative to the bio-sample 112 in the test information 132. In step S1330, the processor 210 acquires gut management information 1060 related to the determined enterotype from the memory 220.

In one embodiment, the method 1300 further includes transmitting, by the processor 210, information indicating the enterotype and/or the gut management information 1060 to the device 140 of the subject by using the communication circuit 230.

In one embodiment, the method 1300 further includes determining, by the processor 210, the enterotype as the first type 1010 if the level of Proteobacteria in the bio-sample 112 exceeds approximately 10% or the level of *Fusobacterium* in the bio-sample 112 exceeds approximately 1%.

In one embodiment, the method 1300 further includes determining, by the processor 210, the gut microbiome index indicating the state of the gut microbiome of the subject 110 on the basis of the test information 132, and/or determining the enterotype as the first type 1010 on the basis of the level of the inflammation-specific biomarker relative to the bio-sample 112 and the gut microbiome index.

In one embodiment, the method 1300 further includes determining, by the processor 210, determining the enterotype as the first type 1010 if the level of the inflammation-specific biomarker relative to the bio-sample 112 satisfies a predetermined first standard or the gut microbiome index satisfies a predetermined second standard.

In one embodiment, the method 1300 further includes determining, by the processor 210, first, second, third, and/or fourth information on the basis of the test information 132, and/or determining the gut microbiome index on the basis of the first, second, third, and/or fourth information.

In one embodiment, the method 1300 further includes determining, by the processor 210, the gut microbiome index by applying predetermined weights $c_1$, $c_2$, $c_3$, and $c_4$ to the first, second, third, and/or fourth information, respectively.

In one embodiment, the method 1300 further includes transmitting, by the processor 210, the information indicating the enterotype, the gut management information 1060, and/or the information 136 indicating the gut microbiome index to the device 140 of the subject by using the communication circuit 230.

In one embodiment, the method 1300 further includes, when the enterotype of the subject 110 is not determined as the first type 1010, determining, by the processor 210, the enterotype as the second type 1020 if the level of Prevotella relative to the bio-sample 112 exceeds approximately 3% or determining the enterotype as the third type 1030 if does not exceed approximately 3%.

The methods according to the present disclosure are computer implemented methods. In the present disclosure, respective steps of the corresponding methods are shown and described in sequence, but the respective steps are performed not only in sequence but also in an order in which the steps can be arbitrarily combined according to the present disclosure. In one embodiment, at least some steps are performed in parallel, repeatedly, or heuristically. The present disclosure is not intended to exclude a change or modification to the corresponding methods. In one embodiment, at least some of the steps are omitted, or other steps are added.

Various embodiments of the present disclosure are implemented as software in a machine-readable recoding medium. The software is software for implementing various embodiments of the present disclosure described above. The software is inferred from various embodiments of the present disclosure by programmers of the technical field to which this disclosure belongs. For example, the software is machine-readable commands (e.g., codes or code segments) or programs. The machine is an apparatus operable according to a commands called from a storage medium, and is, for example, a computer. In one embodiment, the machine is the inspection apparatus 100 according to embodiments of the present disclosure. In one embodiment, a processor of the machine executes the called commands so as to cause the components of the machine to perform functions corresponding to the corresponding commands. In one embodiment, the processor 210 is the processor according to embodiments of the present disclosure. The recoding medium includes any kind of data storage devices that can be read by a machine. Examples of the recording medium include ROM, RAM, CD-ROM, magnetic tape, floppy disk, optical data storage device and the like. In one embodiment, the recording medium is the memory 220. In one embodiment, the recoding medium is implemented in a distribution manner to computer systems which are connected through a network. The software is stored and executed in computer systems and the like. The recording medium is a non-transitory recording medium. The non-transitory recording medium means a tangible medium regardless of whether data is stored therein semi-permanently or temporarily, and does not include a signal propagated in a transitory manner.

**[Customized health functional food suited to enterotype]**

In an aspect of the present disclosure, a health functional food composition to be provided for a subject is proposed. In one embodiment, the health functional food composition is a customized health functional food composition, and the health functional food composition is provided for subjects classified as a specific enterotype. The specific enterotype is determined by an apparatus or method according to one embodiment of the present disclosure, and is, for example, a first type, a second type, and/or a third type according to one embodiment of the present disclosure, is a type resulting from sub-classification thereof. In one embodiment, the health functional food composition can increase the gut microbiome index of the subject. In one embodiment, the health functional food composition means a food that is manufactured and processed using raw materials or components having functionality useful for the human body. Such a health functional food composition contains additional components (e.g., dietary fibers with prebiotic activity, such as fructo-oligosaccharides, inulin, and partially hydrolyzed guar gum (PHGG)), which a person skilled in the art considers for manufacturing and distributing the corresponding composition, and especially, contains prebiotics that can help the proliferation of probiotics ingested together.

In one embodiment, the prebiotics are one or more of those recited on Tables 3 and 4 below.

TABLE 3

| | | | |
|---|---|---|---|
| Aspartame | Linseed powder | Cinnamon powder | Chicory fibre |
| Sorbitol | Safflower seeds | Onion powder | Inulin |
| MSG | Soybean powder | Wheat flour | Polydextrose |
| Ginseng powder | Licorice powder | Glutinous rice flour | Soybean oligosaccharides |
| Milk powder | Anchovy powder | Bread powder | Isomaltooligosaccharides |
| Red ginseng powder | Garlic powder | Red pepper powder | Gluco-oligosaccharides |
| Konjac powder | Spinach powder | Black pepper | Xylo-oligosaccharides |
| Acai Berry powder | Dried Pollack powder | Aloe powder | Palatinose |
| Protein supplement | Shrimp powder | Honey | Gentio-oligosaccharides |
| Chicory root extract powder | Mussel powder | Prune juice | Some starch derivatives |
| Roasted adzukin powder | Fish collagen powder | Saccharin | Lactitol |
| Chaga mushroom powder | Beer yeast powder | Mannitol | Sorbitol |
| Broccoli powder | Autumn squash powder | Mucin | Maltitol |
| Almond powder | Purple sweet potato powder | Glucose | Sugar alcohols |
| Aronia powder | Jerusalem artichoke powder | Fructose | Human milk oligosaccharides (hmos) |
| Roasted black bean powder | Kelp powder | Galactose | Pullulan |

(continued)

| Chlorella | Coconut oil | Fucose | Mannan-oligosaccharides (MOS) |
|---|---|---|---|
| Mixed grain powder | Lotus root powder | Mannose | B-glucan |
| Peanut powder | White bean powder | Pyruvate | D-mannose |
| Perilla powder | Roasted brown rice powder | Sucrose | Galactosamine |
| GNC omega fish body oil | Cocoa powder | Lactose | Refined functional carbohydrates (RFC) |
| Balloonflower root powder | Puer tea extract powder | Maltose | Lactosucrose |
| Shiitake mushroom powder | Noni powder | Trehalose | Tagatose |
| Green apple powder | Rice germ powder | Fructo-oligosaccharides | Lactulose |
| Ginger powder | Xylitol powder | Mannan-oligosaccharides | Lactitol |
| Roasted oat powder | Gelatin powder | Starch | Lactobionic acid |
| Curcuma powder | Acorn powder | Cellulose | Lactose-derivatives |
| Cabbage powder | Rice flour | Galacto-oligosaccharides (GOS) | Disaccharide lactose |
| Cheonggukj ang powder | Coffee powder | Fructo-oligosaccharides (FOS) | Prebiotic oligosaccharides (OS) |
| Sprout barley powder | Hibiscus powder | Oligofructose (OF) | Polydextrose (PDX) |

TABLE 4

| Resistant starch | Isomaltooligosides | Lactitol | Rebaudioside a,b,c,d,e,f |
|---|---|---|---|
| Feruloylated arabinoxylan oligosaccharides (faxo) | Oligolaminarans (β-glucans) | Mannitol | Dulcoside a,b |
| Polyphenols (rbpp) | D-tagatose | Rebaudiosides | Rubusoside |
| Ellagitannins (ets) | Saccharin (sweet 'n low) | Steviolbioside | Mogroside iv, v |
| Proanthocyanidins (pac) | Acesulfame | Dulcosides | Siamenoside |
| Indigestible dextrin (dex) | Aspartame (nutrasweet or equal) | Glycoside derivatives | Monatin and salts thereof (monatin ss, rr, rs, sr) |
| A-cyclodextrin (αcd) | Neotame | Salt of aspartame-acesulfame | Hernandulcin |
| Dextran (dxr) | Sucralose (splenda) | Neohesperidine dc | Phyllodulcin |
| Short-chain fructo-oligosaccharide (scfos) | Stevia | Advantame | Glycyphyllin |
| Short chain galacto oligosaccharides (scgos) | Erythritol | Rebaudioside a | Phloridzin |
| Long chain fructo oligosaccharides (lcfos) | Xylitol | Tagatose | Trilobatin |

(continued)

| Glutamine | Yacon syrup | Trehalose | Baiyunoside |
|---|---|---|---|
| Inulin-type fructans | Coconut sugar | Corn sweetener | Osladine |
| Cyclodextrins (cds) | Molasses | Sucrose (table sugar) | Polypodoside a |
| Acidic oligosaccharides (aos) | Acesulfame potassium (sunett) | Monk fruit extracts (luo han guo sweetener) | Pterocaryoside a |
| Cellulose | Acesulfame k | High fructose corn syrup (hfcs) | Pterocarioside b |
| Hemicellulose | D-tagatose (sugaree) | Monellin | Mocurozioside |
| Pectins | Sorbitol | Mabinlin | Name |
| Gums | Steviol glycosides | Pentadin | Phlomisosode i |
| Guar gum | Cyclamate | Curculin | Periandrin i |
| Maltodextrin | Alitame | Miraculin | Abrusoside a |
| Arabianooligosaccharides | Talin | Cyclamate sodium (not sold in the us) | Cyclocarioside i |
| Psyllium | Thaumatin | M-erythritol | |
| Acacia gum | Brazzein | D-fructose | |
| Levans | Stevioside | D-glucose | |
| Lactulose | Glycyrrhizinic acid | D-solbitol | |
| (trans)galacto-oligosides (tos) | Mogroside | Ribitol (adonitol) | |
| Galacto-oligosides (gos) | Neohesperidin | Sodium saccharin | |
| Soyoligosides | Maltitol | L-aspartyl-1-phenylalanine methyl ester (apm) | |
| A-gal actosi des (raffinose, s tachyose&verbascose) | Isomalt | Natural high potency sweetener (nhps) | |

In one embodiment, prebiotics mean nondigestible food components that can improve human health by selectively stimulating the growth and activity of one or more probiotic microbes in the gut. The prebiotics are non-digestible carbohydrates (oligo- or poly-saccharides) or sugar alcohols that are not broken down or absorbed in the upper digestive tract. For example, inulin, fructo-oligosaccharides, xylo-oligosaccharides, or transgalacto-oligosaccharides is used.

In one embodiment, the health functional food composition contains probiotics including one or more microbes described to be provided for the subject in one embodiment of the present disclosure. In one embodiment, the health functional food composition includes a combination of one or more microorganisms described as being provided to a subject in one embodiment of the present disclosure.

In one embodiment, there can be provided a customized health functional food composition to be provided for a subject classified as the first type, the composition containing at least one microbe selected from the group consisting of Lactobacillus paracasei subsp. tolerance HM0866, Lactobacillus fermentum HM0740, Lactobacillus plantarum subsp. plantarum HM0782, and Lactococcus lactis subsp. lactis HM0850.

In one embodiment, there can be provided a customized health functional food composition to be provided for a subject classified as the second type, the composition containing at least one microbe selected from the group consisting of Lactobacillus fermentum HM0740 and Lactococcus lactis subsp. lactis HM0850.

In one embodiment, there can be provided a customized health functional food composition to be provided for a subject classified as the third type, the composition containing at least one microbe selected from the group consisting of Lactobacillus fermentum HM0740, Lactobacillus plantarum subsp. plantarum HM0782, and Lactococcus lactis subsp. lactis HM0850.

Although the technical idea of the present disclosure has been described by various embodiments, it should be noted that various substitutions, modifications, and changes can be made within a range that can be understood by those skilled in the art to which the present disclosure pertains. In addition, it should be noted that that such substitutions, modifications and changes are intended to fall within the scope of the appended claims. The embodiments according to the present disclosure are combined with each other. The embodiments are variously combined according to the number of cases, and the combined embodiments are also within the scope of the present disclosure.

[Test Example 1]

1. Bio-sample collection

The second citizen science project (IRB No. P01-2019-05-11-004, Human Study approved by Institutional Review Board designated by the Ministry of Health and Welfare) by a service provider, which is the present applicant, recruited willing volunteers from Korean healthy men and women aged 19 years or older and selected approximately 1081 test subjects therefrom. Each subject was given a stool collection kit, which was composed of a stool collection case, a stool collection paper, and an instruction manual, and enclosed with study instructions and consent form.
Among the selected subjects, 452 subjects were allowed to ingest different microbial strains on Table 5 below for two weeks, and the bio-samples of the subjects were collected before and after ingestion and analyzed for the gut microbiome index.

TABLE 5

| Assignment No. | Strain |
|---|---|
| A | *Lactobacillus paracasei subsp. tolerance* HM0866 (accession number: KCTC14409BP) |
| B | *Lactobacillus fermentum* HM0740 (accession number: KCTC14406BP) |
| C | *Lactobacillus plantarum subsp. plantarum* HM0782 (accession number: KCTC14407BP) |
| D | *Lactococcus lactis subsp. lactis* HM0850 (accession number: KCTC14408BP) |

The stool of each subject was delivered in a buffer preventing microbial degeneration. The composition of the buffer is shown in Table 6. The stool collection kit after stool collection was recommended to be sent 48 hours before stool collection, and guided to be stored at room temperature in a cool place out of sunlight to prevent sample deterioration until the time of sending.

TABLE 6

| Component | Final concentration | Product name |
|---|---|---|
| SDS | 4% | 10 % Sodium Dodecyl Sulfate (Sigma, Cat. No. 71736-500 ML) |
| Tris-HCl | 50 mM | 1 M Tris-HCl, pH 8.0 (BIOSESANG, Cat. No. T2016-8.0) |
| EDTA | 50 mM | 500 mM Ethylenediamine Tetraacetic acid, pH 8.0 (BIOSESANG, Cat. No. E2002) |
| NaCl | 500 mM | 5 M Sodium chloride (Sigma, Cat. No. 71386-1L) |

2. Gut microbial community analysis

(1) 16S ribosomal RNA gene sequencing

Stool genomic DNA was extracted using the collected stool samples. Since each sample was received in a DNA buffer, genomic DNA was physically extracted by homogenization with, specifically, a FastPrep (MP Biomedicals) at a speed of 6.0 for 40 seconds immediately after receipt.
Specifically, various types of amplicons for a wide range of taxonomic groups were formed through a polymerase chain reaction (PCR) using universal primers along with the DNA extracted by genomic DNA extraction as above. The sequences of the universal primers were as follows, and the PCR premix composition and PCR conditions for amplicon formation are shown in Tables 7 and 8, respectively.

Forward universal primer (SEQ ID NO: 75): 5-CCTACGGGNGGCWGCAG-3'

Reverse universal primer (SEQ ID NO: 76): 5-GACTACHVGGGTATCTAATCC-3'

TABLE 7

| Composition | Content (1X) |
|---|---|
| template (genomic DNA) | 0.5 µl |
| 2x buffer | 10 µl |
| universal primer 1 (10 pmole) | 0.5 µl |
| universal primer 2 (10 pmole) | 0.5 µl |
| Polymerase | 0.3 µl |
| 3' D.W | 8.2 µl |
| Total | 20 µl |

TABLE 8

| Stage | Temperature | Time |
|---|---|---|
| Initial denaturation | 95°C | 3 min |
| Denaturation annealing& | 95°C | 30 sec |
| Extension (25 cycles) | 55°C<br>72°C | 30 sec<br>30 sec |
| Final extension | 72°C<br>4°C | 5 min<br>∞ |

The amplicons thus formed were purified and then subjected to amplicon quality control (QC) using the Bioanalyzer (Agilent), qPCR, and the like, to confirm that gut microbial 16S rRNA sequences extracted from the stool genomic DNA. Thereafter, the 16s ribosomal RNA gene sequences of the samples were sequenced using next generation sequencing (NGS) through the MiSeq (Illumina) system. Sample pre-treatment and QC include sample preparation (sample dilution and dissolution), DNA extraction (cell lysis and DNA extraction), DNA amplification using PCR, pooing (sample mixing), and uhm sequencing (sequencing using an NGS system).

Specifically, it was investigated in the DNA amplification process whether DNA bands were observed at around 650 bp through Gel QC results, and the DNA concentration was set to 5 ng/µl or more as a result of DNA quantitative analysis using PicoGreen reagent. It was investigated whether short peaks other main peaks were observed on DNA peaks through the Bioanalyzer QC results, and quality control was performed at a DNA concentration of 5 ng/µl or more as a PicoGreen QC results.

(2) Microbial community analysis

Thousands of gene sequences were generated from one sample by the next-generation sequencing technique (NGS), and then the microbial community (bacterial community) information was analyzed from phylum to species levels by 16S ribosomal RNA gene sequence database (EzTaxon) of standard strains and non-cultured microorganisms and the EzBioCloud analysis system (http://www.ezbiocloud.com).

3. Enterotype analysis

Through the microbial community analysis data of each subject analyzed in section 2. above, the enterotype of the subject was analyzed and classified as a first type, a second type, and a third type by using the first process and the second process according to one embodiment of the present disclosure. The results of analyzing the enterotype of each subject ingesting the respective strains are shown in Table 9 below.

TABLE 9

| Strain assignment No | First type (n) | Second type (n) | Third type (n) |
|---|---|---|---|
| A | 12 | 35 | 56 |
| B | 23 | 47 | 59 |
| C | 15 | 49 | 47 |
| D | 6 | 44 | 56 |

4. Gut microbiome index analysis

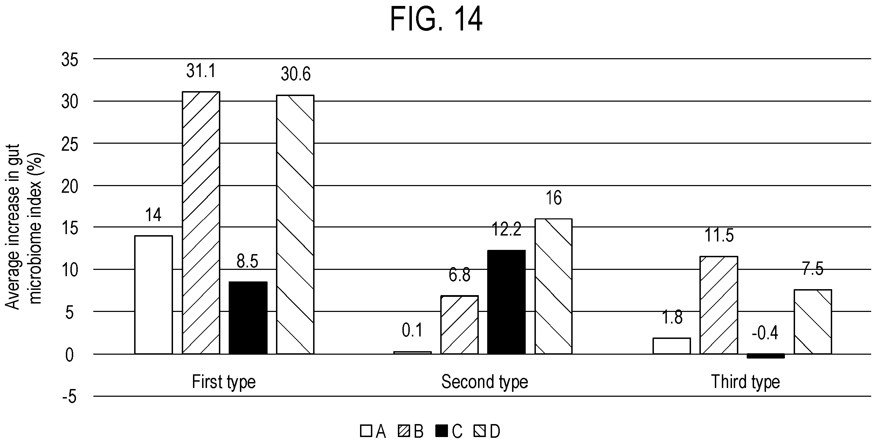

Through the microbial community analysis data of the subject analyzed in section 2. above, the gut microbiome index was analyzed by a method according to one embodiment of the present disclosure. To investigate an increase or decrease in gut microbiome index, samples were selected according to the corresponding index, and an average change in gut microbiome index before and after ingestion of strains was calculated and shown in FIG. 14.

Samples with a gut microbiome index of less than 70 points were selected for the second type, and samples a gut microbiome index of less than 60 points were selected for the first type and the third type. The selection results are shown below.

TABLE 10

| Strain assignment No. | First type (n) | Second type (n) | Third type (n) |
|---|---|---|---|
| A | 12 | 19 | 11 |
| B | 23 | 25 | 23 |
| C | 15 | 29 | 14 |
| D | 6 | 14 | 10 |

Specifically, the gut microbiome index was calculated using the function shown in FIG. 7, wherein the first information was calculated according to Equation 1; the second information was calculated according to Equation 4; the third information was calculated according to Equation 5; and the fourth information was calculated according to Equation 6. Referring to FIG. 14, the subjects with the first type showed that the gut microbiome index was increased by 5% or more for all of the strains A, B, C, and D. Particularly, the gut microbiome index was increased by approximately 30% for the stains B and D. Therefore, the subjects with the first type are suggested to ingest the strains A to D to increase the gut microbiome index, and customized health functional foods containing at least one of these strains will increase the gut microbiome index of the eaters. Furthermore, a combination of these strains will further increase the gut microbiome index when suggested or ingested.

The subjects with the second type showed that the gut microbiome index was increased by 5% or more when ingesting the strains B and D. Therefore, the subjects with the second type are suggested to ingest the strains B and D to increase the gut microbiome index, and customized health functional foods containing at least one of these strains will increase the gut microbiome index of the eaters. Furthermore, a combination of these strains will further increase the gut microbiome index when suggested or ingested.

The subjects with the third type showed that the gut microbiome index was increased by 5% or more when ingesting the strains B, C, and D. Therefore, the subjects with the third type are suggested to ingest the strains B, C, and D to increase the gut microbiome index, and customized health functional foods containing at least one of these strains will increase the gut microbiome index of the eaters. Furthermore, a combination of these strains will further increase the gut microbiome index when suggested or ingested.

[Accession number]

Depository institution name: Korea Research Institute of Bioscience and Biotechnology
Accession number: KCTC 14406BP
Deposit date: 20201210
Depository institution name: Korea Research Institute of Bioscience and Biotechnology

Accession number: KCTC14407BP
Deposit date: 20201210
Depository institution name: Korea Research Institute of Bioscience and Biotechnology
Accession number: KCTC 14408BP
Deposit date: 20201210
Depository institution name: Korea Research Institute of Bioscience and Biotechnology
Accession number: KCTC 14409BP
Deposit date: 20201210

**Claims**

1.  An apparatus comprising:

    a communication circuit;
    at least one processor; and
    at least one memory configured to store instructions that cause the at least one processor to perform an operation when the instructions are executed by the at least one processor,
    wherein the at least one processor is configured to:

    acquire test information about a bio-sample of a subject from at least one test apparatus, by using the communication circuit;
    based on the test information, determine first information about microbial similarity between a predetermined reference sample and the bio-sample, second information about proportions of harmful gut microbes of the subject, third information about proportions of beneficial gut microbes of the subject, and fourth information about gut microbial diversity of the subject; and
    based on the first information, the second information, the third information, and the fourth information, determine a gut microbiome index indicating a state of a gut microbiome of the subject.

2.  The apparatus of claim 1, wherein the at least one processor is configured to transmit information indicating the gut microbiome index of the subject to a device by using the communication circuit.

3.  The apparatus of claim 1, wherein the at least one memory is configured to further store information about the reference sample, and
    wherein the at least one processor is configured to:

    based on the test information and the information about the reference sample, determine similarity between a distribution of a presence proportion of each microbial species in the bio-sample and a distribution of a presence proportion of each microbial species in the reference sample; and
    based on the similarity, determine the first information.

4.  The apparatus of claim 1, wherein the at least one processor is configured to:

    based on the test information, determine, relative to predetermined commensal microbial species and harmful microbial species in the bio-sample, presence proportions of the harmful microbial species; and
    based on the presence proportions, determine the second information.

5.  The apparatus of claim 1, wherein the at least one processor is configured to:

    based on the test information, determine presence proportions of predetermined beneficial microbial species in the bio-sample; and
    based on the presence proportions, determine the third information.

6.  The apparatus of claim 1, wherein the at least one processor is configured to:

    based on the test information, determine a number of all microbial species in the bio-sample and a degree to which a distribution of a presence proportion of each of all the microbial species are even; and
    based on the number of all the microbial species and a degree of evenness, determine the fourth information.

7. The apparatus of claim 1, wherein the at least one processor is configured to determine the gut microbiome index by applying predetermined weights to the first information, the second information, the third information, and the fourth information, respectively.

8. A method performed by an apparatus comprising at least one processor and at least one memory storing instructions executed by the at least one processor, the method comprising:

   acquiring, by the at least one processor, test information about a bio-sample of a subject from at least one test apparatus;
   based on the test information, determining, by the at least one processor, first information about microbial similarity between a predetermined reference sample and the bio-sample, second information about proportions of harmful gut microbes of the subject, third information about proportions of beneficial gut microbes of the subject, and fourth information about gut microbial diversity of the subject; and
   based on the first information, the second information, the third information, and the fourth information, determining, by the at least one processor, a gut microbiome index indicating a state of a gut microbiome of the subject.

9. A non-transitory computer-readable recording medium storing instructions that cause at least one processor to perform an operation when the instructions are executed by the at least one processor, wherein the instructions cause the at least one processor to:

   based on test information about a bio-sample of a subject that is acquired from at least one test apparatus, determine first information about microbial similarity between a predetermined reference sample and the bio-sample, second information about proportions of harmful gut microbes of the subject, third information about proportions of beneficial gut microbes of the subject, and fourth information about gut microbial diversity of the subject; and
   based on the first information, the second information, the third information, and the fourth information, determine a gut microbiome index indicating a state of a gut microbiome of the subject.

10. An apparatus comprising:

    a communication circuit;
    at least one processor; and
    at least one memory configured to store instructions that cause the at least one processor to perform an operation when the instructions are executed by the at least one processor,
    wherein the at least one processor is configured to:

    acquire test information about a bio-sample of a subject from at least one test apparatus, by using the communication circuit;
    determine an enterotype of the subject as a first type, based on: a level of an inflammation-specific biomarker relative to the bio-sample in the test information; and/or a gut microbiome index indicating a state of a gut microbiome of the subject and determined based on the test information; and
    acquire gut management information related to the enterotype from the at least one memory,
    wherein the gut management information indicates gut microbiome regulating suggestions for the subject with the enterotype.

11. The apparatus of claim 10, wherein the at least one processor is configured to transmit information indicating the enterotype and the gut management information to a device of the subject, by using the communication circuit.

12. The apparatus of claim 10, wherein the inflammation-specific biomarker is at least one microbiome selected from phylum Proteobacteria microbes and genus *Fusobacterium* microbes.

13. The apparatus of claim 12, wherein the at least one processor is configured to determine the enterotype as the first type if a presence proportion of the phylum Proteobacteria microbes relative to the bio-sample exceeds 10% or a presence proportion of the genus *Fusobacterium* microbes relative to the bio-sample exceeds 1%.

14. The apparatus of claim 10, wherein the at least one processor is configured to determine the enterotype as the first type if the level of the inflammation-specific biomarker relative to the bio-sample satisfies a predetermined first standard and/or the gut microbiome index satisfies a predetermined second standard.

**15.** The apparatus of claim 10, wherein the at least one processor is configured to:

based on the test information, determine first information about microbial similarity between a predetermined reference sample and the bio-sample, second information about proportions of harmful gut microbes of the subject, third information about proportions of beneficial gut microbes of the subject, and fourth information about gut microbial diversity of the subject; and
based on the first information, the second information, the third information, and the fourth information, determine the gut microbiome index.

**16.** The apparatus of claim 15, wherein the at least one processor is configured to determine the gut microbiome index by applying predetermined weights to the first information, the second information, the third information, and the fourth information, respectively.

**17.** The apparatus of claim 10, wherein the at least one processor is configured to transmit information indicating the the enterotype, the gut management information, and information indicating the gut microbiome index to a device of the subject by using the communication circuit.

**18.** The apparatus of claim 10, wherein the at least one processor is configured to, when the enterotype of the subject is not determined as the first type, determine the enterotype as a second type if a presence proportion of genus Prevotella microbes relative to the bio-sample exceeds 3%, and determine the enterotype as a third type if does not exceed 3%.

**19.** The apparatus of claim 10, wherein the gut microbiome regulating suggestions of the gut management information include at least one selected from ingesting specific probiotics corresponding to the enterotype, ingesting a specific food, and applying a specific life habit.

**20.** The apparatus of claim 19, wherein the gut microbiome regulating suggestions of the gut management information have priority according to a degree to which each of the suggestions changes the gut microbiome index of the subject with the enterotype.

**21.** The apparatus of claim 19, wherein the gut microbiome regulating suggestions include:

(i) suggesting, to a subject classified as the first type, probiotics containing at least one species of microbes including at least one *Lactobacillus paracasei*, at least one *Lactobacillus fermentum*, at least one *Lactobacillus plantarum* group, and/or at least one *Lactococcus lactis* group;
(ii) suggesting, to a subject classified as a second type 1020, probiotics containing at least one species of microbes including at least one *Lactobacillus fermentum* and/or at least one *Lactococcus lactis* group; or
(iii) suggesting, to a subject classified as a third type 1030, probiotics containing at least one species of microbes including at least one at least one *Lactobacillus fermentum,* at least one *Lactobacillus plantarum* group, and/or at least one *Lactococcus lactis* group.

**22.** The apparatus of claim 21, wherein the at least one *Lactobacillus paracasei* includes *Lactobacillus paracasei subsp. paracasei*, *Lactobacillus paracasei subsp. tolerance*, or *Lactobacillus paracasei subsp. tolerance* HM0866 (accession number: KCTC14409BP).

**23.** The apparatus of claim 21, wherein the at least one *Lactobacillus fermentum* includes *Lactobacillus fermentum* HM0740 (accession number: KCTC14406BP).

**24.** The apparatus of claim 21, wherein the at least one *Lactobacillus plantarum* group includes at least one selected from a group consisting of *Lactobacillus plantarum*, *Lactobacillus pentosus*, *Lactobacillus paraplantarum*, *Lactobacillus fabifermentans*, *Lactobacillus xiangfangensis*, *Lactobacillus herbarum*, *Lactobacillus modestisalitolerans*, and subspecies thereof.

**25.** The apparatus of claim 24, wherein the *Lactobacillus plantarum* includes *Lactobacillus plantarum subsp. plantarum*, *Lactobacillus plantarum subsp. argentoratensis,*, or *Lactobacillus plantarum subsp. plantarum* HM0782 (accession number: KCTC14407BP).

**26.** The apparatus of claim 21, wherein the at least one *Lactococcus lactis* group includes at least one selected from a group consisting of *Lactococcus lactis subsp. lactis*, *Lactococcus lactis subsp. hordniae*, *Lactococcus lactis subsp. cremoris*, *Lactococcus lactis subsp. tructae*, and *Lactococcus lactis subsp. lactis* HM0850 (accession number: KCTC14408BP).

27. The apparatus of claim 21, wherein the at least one *Lactobacillus paracasei* includes *Lactobacillus paracasei subsp. tolerance* HM0866 (accession number: KCTC14409BP),

   wherein at least one *Lactobacillus fermentum* includes *Lactobacillus fermentum* HM0740 (accession number: KCTC14406BP),
   wherein at least one *Lactobacillus plantarum* includes *Lactobacillus plantarum subsp. plantarum* HM0782 (accession number: KCTC14407BP), and
   wherein the at least one *Lactococcus lactis* group includes *Lactococcus lactis subsp. lactis* HM0850 (accession number: KCTC14408BP).

28. A method performed by an apparatus comprising at least one processor and at least one memory storing instructions executed by the at least one processor, the method comprising:

   acquiring, by the at least one processor, test information about a bio-sample of a subject from at least one test apparatus;
   determining, by the at least one processor, an enterotype of the subject as a first type, based on a level of an inflammation-specific biomarker relative to the bio-sample in the test information; and
   acquiring, by the at least one processor, gut management information related to the enterotype from the at least one memory,
   wherein the gut management information indicates gut microbiome regulating suggestions for the subject with the enterotype.

29. A non-transitory computer-readable recording medium storing instructions that cause at least one processor to perform an operation when the instructions are executed by the at least one processor, wherein the instructions cause the at least one processor to:

   determine an enterotype of a subject as a first type, based on a level of an inflammation-specific biomarker relative to a bio-sample, in test information about the bio-sample of the subject acquired from at least one test apparatus; and
   acquire gut management information related to the enterotype from at least one memory,
   wherein the gut management information indicates gut microbiome regulating suggestions for the subject with the enterotype.

# FIG. 1

Bio-sample (112)

Service provider (120)

Subject (110)

Test apparatus (130)

Device of subject (140)

Storing information (134) about reference samples (114) and the like

Apparatus (100)

# FIG. 2

Processor ~210

Communication circuit ~230

BUS

Memory ~220

Input/Output interface ~220

100

# FIG. 3

310

| Microbial similarity-related index | Mean Balanced Accuracy | Number of Significances |
|---|---|---|
| -avg_jsd_ctl | 0.66983 | 14 |
| -avg_braycurtis_ctl | 0.662275 | 13 |
| +avg_aitchison_case | 0.650174 | 12 |
| -avg_aitchison_ctl | 0.613557 | 7 |
| +avg_jsd_case | 0.58427 | 2 |
| -avg_braycurtis_case | 0.571717 | 5 |
| +avg_braycurtis_case | 0.566254 | 2 |
| -avg_jsd_case | 0.563281 | 5 |
| +avg_aitchison_ctl | 0.534596 | 0 |
| -avg_aitchison_case | 0.526884 | 0 |
| +avg_braycurtis_ctl | 0.525166 | 0 |
| +avg_jsd_ctl | 0.520659 | 0 |

# FIG. 4

410

| Harmful microbe-related index | Mean Balanced Accuracy | Number of Significances |
|---|---|---|
| -dysbiosis_index | 0.640219 | 10 |
| +hrm_sp_shannon | 0.608821 | 5 |
| +hrm_sp_invsimpson | 0.603572 | 6 |
| +hrm_sp_shannon_e | 0.603091 | 5 |
| -hrm_sp_berger_parker_d | 0.601678 | 6 |
| +hrm_sp_heip_e | 0.599442 | 6 |
| -hrm_sum | 0.59688 | 7 |
| +hrm_sp_simpson_e | 0.578927 | 4 |
| +hrm_sp_alatalo_e | 0.576994 | 3 |
| +hrm_sp_numotu | 0.574144 | 4 |
| -hrm_sp_alatalo_e | 0.561965 | 3 |
| +hrm_sp_berger_parker_d | 0.552615 | 1 |
| -hrm_sp_numotu | 0.545141 | 2 |
| -hrm_sp_invsimpson | 0.543901 | 1 |
| -hrm_sp_shannon | 0.543828 | 1 |
| -hrm_sp_simpson_e | 0.543012 | 0 |
| +hrm_sum | 0.53728 | 0 |
| -hrm_sp_heip_e | 0.535262 | 1 |
| -hrm_sp_shannon_e | 0.534504 | 1 |
| +dysbiosis_index | 0.521336 | 0 |

# FIG. 5

510

| Beneficial microbe-related index | Mean Balanced Accuracy | Number of Significances |
|---|---|---|
| +ben_sp_numotu | 0.656145 | 13 |
| +ben_sp_shannon | 0.652678 | 13 |
| +ben_sum | 0.645077 | 11 |
| +ben_sp_invsimpson | 0.643184 | 12 |
| -ben_sp_berger_parker_d | 0.62718 | 11 |
| +ben_sp_shannon_e | 0.615283 | 9 |
| -ben_sp_simpson_e | 0.587812 | 4 |
| +ben_sp_heip_e | 0.580154 | 5 |
| -ben_sp_alatalo_e | 0.568943 | 2 |
| +ben_sp_alatalo_e | 0.558876 | 2 |
| +ben_sp_simpson_e | 0.558638 | 1 |
| -ben_sp_heip_e | 0.557801 | 2 |
| -ben_sp_shannon_e | 0.537911 | 1 |
| +ben_sp_berger_parker_d | 0.528473 | 0 |
| -ben_sum | 0.523317 | 0 |
| -ben_sp_invsimpson | 0.515793 | 0 |
| -ben_sp_numotu | 0.514095 | 0 |
| -ben_sp_shannon | 0.512274 | 0 |

# FIG. 6

610

| Microbial diversity-related index | Mean Balanced Accuracy | Number of Significances |
|---|---|---|
| +shannon | 0.642489 | 12 |
| +numotu | 0.641521 | 10 |
| +invsimpson | 0.641519 | 13 |
| +shannon_e | 0.639557 | 11 |
| -berger_parker_d | 0.632814 | 12 |
| +heip_e | 0.618792 | 7 |
| +simpson_e | 0.582701 | 4 |
| -alatalo_e | 0.5651 | 0 |
| +alatalo_e | 0.564951 | 4 |
| -simpson_e | 0.541983 | 1 |
| -heip_e | 0.53291 | 0 |
| +berger_parker_d | 0.530816 | 0 |
| -shannon_e | 0.528736 | 0 |
| -invsimpson | 0.524885 | 0 |
| -numotu | 0.523036 | 0 |
| -shannon | 0.517932 | 0 |

# FIG. 7

Gut microbiome index
= logit[(c1 X first information) + (c2 X second information) +(c3 X third information) +(c4 X fourth information)]

# FIG. 8

## ROC Curve

# FIG. 9

Precision-Recall Curve

# FIG. 10

Subject (110)

Device of subject (140)

Apparatus (100)

<Enterotype determining process>

Second type (1020)　　Third type (1030)

Bio-sample (112)

First type (1010)

- First process (1041)
- Second process (1042)
- Third process (1043)

# FIG. 11

Gut management information related to first type (1110)

| 1st | Ingesting nuts | ingesting handful of mixed nuts of walnuts, almonds, and peanuts daily |
|---|---|---|
| 2nd | Ingesting vegetables | consistently ingesting my customized dietary fibers 1-3 times daily |
| 3rd | Exercising | consistently exercising 1-2 times a day for at least 30 minutes each time |
| 4th | Ingesting yogurt | consistently ingesting scoopable yogurt |
| 5th | Ingesting probiotics | being sure to check health functional food certification mark |
| 6th | Ingesting brown rice | changing daily diet from white rice to at least 40% of brown rice |
| 7th | Ingesting fruits | consistently ingesting fruits 1-2 times daily |

Gut management information related to second type (1120)

| 1st | Ingesting yogurt | consistently ingesting scoopable yogurt |
|---|---|---|
| 2nd | Ingesting nuts | ingesting handful of mixed nuts of walnuts, almonds, and peanuts daily |
| 3rd | Ingesting brown rice | changing daily diet from white rice to at least 40% of brown rice |
| 4th | Ingesting fruits | consistently ingesting fruits 1-2 times daily |
| 5th | Exercising | consistently exercising 1-2 times a day for at least 30 minutes each time |
| 6th | Ingesting vegetables | consistently ingesting my customized dietary fibers 1-3 times daily |
| 7th | Ingesting probiotics | being sure to check health functional food certification mark |

Gut management information related to third type (1130)

| 1st | Ingesting fruits | consistently ingesting fruits 1-2 times daily |
|---|---|---|
| 2nd | Ingesting brown rice | changing daily diet from white rice to at least 40% of brown rice |
| 3rd | Ingesting nuts | ingesting handful of mixed nuts of walnuts, almonds, and peanuts daily |
| 4th | Ingesting yogurt | consistently ingesting scoopable yogurt |
| 5th | Exercising | consistently exercising 1-2 times a day for at least 30 minutes each time |
| 6th | Ingesting probiotics | being sure to check health functional food certification mark |
| 7th | Ingesting vegetables | consistently ingesting my customized dietary fibers 1-3 times daily |

# FIG. 12

Acquiring test information about bio-sample
of subject from test apparatus — S1210

Determining first information, second information,
third information, and/or fourth information on the
basis of test information — S1220

Determining gut microbiome index of subject on
the basis of first information, second information,
third information, and/or fourth information — S1230

# FIG. 13

| |
|---|
| Acquiring test information about bio-sample of subject from test apparatus |

S1310

↓

| |
|---|
| Determining enterotype of subject as first type on the basis of level of inflammation-specific biomarker relative to corresponding bio-sample |

S1320

↓

| |
|---|
| Determining gut management information related to corresponding enterotype from memory |

S1330

FIG. 14

EP 4 167 249 A1

<table>
<tr><td colspan="3" align="center">**INTERNATIONAL SEARCH REPORT**</td><td colspan="2">International application No.<br><br>**PCT/KR2021/007567**</td></tr>
</table>

**A.    CLASSIFICATION OF SUBJECT MATTER**

**G16H 50/70**(2018.01)i; **C12Q 1/6869**(2018.01)i; **G16B 30/00**(2019.01)i; **G16B 40/00**(2019.01)i; **A61B 5/00**(2006.01)i; **G16H 10/20**(2018.01)i

According to International Patent Classification (IPC) or to both national classification and IPC

**B.    FIELDS SEARCHED**

Minimum documentation searched (classification system followed by classification symbols)

G16H 50/70(2018.01); A61K 35/74(2006.01); C12Q 1/689(2018.01); G16B 20/00(2019.01); G16B 30/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 마이크로바이옴(microbiome), 유형(type), 샘플(sample), 분석(analysis), 지시 (instruction)

**C.    DOCUMENTS CONSIDERED TO BE RELEVANT**

| Category* | Citation of document, with indication, where appropriate, of the relevant passages | Relevant to claim No. |
|---|---|---|
| X | KR 10-2020-0051587 A (PSOMAGEN, INC.) 13 May 2020 (2020-05-13)<br>     See paragraphs [0029]-[0145], claims 1 and 11 and figures 1a-2. | 1-29 |
| A | KR 10-2016-0069519 A (THE BOARD OF TRUSTEES OF THE LELAND STANFORD JUNIOR UNIVERSITY) 16 June 2016 (2016-06-16)<br>     See paragraphs [0071]-[0132], claims 1-19 and figure 1i. | 1-29 |
| A | KR 10-2020-0061403 A (REBIOTIX, INC.) 02 June 2020 (2020-06-02)<br>     See paragraphs [0004]-[0063] and claims 1-6. | 1-29 |
| A | WO 2020-037271 A1 (VEDANTA BIOSCIENCES, INC.) 20 February 2020 (2020-02-20)<br>     See entire document. | 1-29 |

☑ Further documents are listed in the continuation of Box C.          ☑ See patent family annex.

| | |
|---|---|
| *    Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **27 September 2021** | **27 September 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

# EP 4 167 249 A1

**INTERNATIONAL SEARCH REPORT**

| International application No. |
| --- |
| **PCT/KR2021/007567** |

### C.    DOCUMENTS CONSIDERED TO BE RELEVANT

| Category* | Citation of document, with indication, where appropriate, of the relevant passages | Relevant to claim No. |
| --- | --- | --- |
|  | 홍숙. 마이크로바이옴이 다가와 말한 '비만'의 비밀. 히트뉴스. [online]. 07 January 2020. non-official translation (HONG, Suk. The Secret Of "Obesity" that Microbiome Said. Hitnews). [Retrieved on 07 September 2021]. Retrieved from <URL:https://www.hitnews.co.kr/news/articleView.html?idxno=13937 >. |  |
| A | See the entire main text. | 1-29 |

Form PCT/ISA/210 (second sheet) (July 2019)

| Patent document cited in search report | | | Publication date (day/month/year) | Patent family member(s) | | | Publication date (day/month/year) |
|---|---|---|---|---|---|---|---|
| KR | 10-2020-0051587 | A | 13 May 2020 | CN | 107075588 | A | 18 August 2017 |
| | | | | CN | 107708714 | A | 16 February 2018 |
| | | | | CN | 107708715 | A | 16 February 2018 |
| | | | | CN | 107708716 | A | 16 February 2018 |
| | | | | CN | 107709576 | A | 16 February 2018 |
| | | | | CN | 107710205 | A | 16 February 2018 |
| | | | | CN | 107835692 | A | 23 March 2018 |
| | | | | CN | 107835859 | A | 23 March 2018 |
| | | | | CN | 107849609 | A | 27 March 2018 |
| | | | | CN | 107849616 | A | 27 March 2018 |
| | | | | CN | 108283012 | A | 13 July 2018 |
| | | | | CN | 108350510 | A | 31 July 2018 |
| | | | | CN | 108472506 | A | 31 August 2018 |
| | | | | CN | 108699586 | A | 23 October 2018 |
| | | | | CN | 109152803 | A | 04 January 2019 |
| | | | | CN | 109475305 | A | 15 March 2019 |
| | | | | CN | 109715059 | A | 03 May 2019 |
| | | | | CN | 111164224 | A | 15 May 2020 |
| | | | | CN | 111164706 | A | 15 May 2020 |
| | | | | CN | 111201323 | A | 26 May 2020 |
| | | | | CN | 111247598 | A | 05 June 2020 |
| | | | | CN | 111315884 | A | 19 June 2020 |
| | | | | CN | 111315898 | A | 19 June 2020 |
| | | | | CN | 111315899 | A | 19 June 2020 |
| | | | | CN | 111465704 | A | 28 July 2020 |
| | | | | EP | 3209803 | A1 | 30 August 2017 |
| | | | | EP | 3283084 | A1 | 21 February 2018 |
| | | | | EP | 3283085 | A1 | 21 February 2018 |
| | | | | EP | 3283086 | A1 | 21 February 2018 |
| | | | | EP | 3283087 | A1 | 21 February 2018 |
| | | | | EP | 3283648 | A1 | 21 February 2018 |
| | | | | EP | 3283649 | A1 | 21 February 2018 |
| | | | | EP | 3283650 | A1 | 21 February 2018 |
| | | | | EP | 3283651 | A1 | 21 February 2018 |
| | | | | EP | 3283652 | A1 | 21 February 2018 |
| | | | | EP | 3283884 | A1 | 21 February 2018 |
| | | | | EP | 3347495 | A1 | 18 July 2018 |
| | | | | EP | 3386592 | A1 | 17 October 2018 |
| | | | | EP | 3426794 | A1 | 16 January 2019 |
| | | | | EP | 3448399 | A1 | 06 March 2019 |
| | | | | EP | 3484348 | A1 | 22 May 2019 |
| | | | | EP | 3512421 | A1 | 24 July 2019 |
| | | | | EP | 3642357 | A1 | 29 April 2020 |
| | | | | EP | 3655971 | A1 | 27 May 2020 |
| | | | | EP | 3669377 | A1 | 24 June 2020 |
| | | | | EP | 3679135 | A1 | 15 July 2020 |
| | | | | EP | 3679162 | A1 | 15 July 2020 |
| | | | | EP | 3682036 | A1 | 22 July 2020 |
| | | | | EP | 3687375 | A1 | 05 August 2020 |
| | | | | EP | 3707280 | A1 | 16 September 2020 |

Form PCT/ISA/210 (patent family annex) (July 2019)

INTERNATIONAL SEARCH REPORT
Information on patent family members

| Patent document cited in search report | Publication date (day/month/year) | Patent family member(s) | | Publication date (day/month/year) |
|---|---|---|---|---|
| | | EP | 3759244 A1 | 06 January 2021 |
| | | EP | 3768851 A1 | 27 January 2021 |
| | | EP | 3769321 A1 | 27 January 2021 |
| | | EP | 3775257 A1 | 17 February 2021 |
| | | EP | 3776285 A1 | 17 February 2021 |
| | | JP | 2019-521706 A | 08 August 2019 |
| | | JP | 2019-528729 A | 17 October 2019 |
| | | JP | 2020-528285 A | 24 September 2020 |
| | | JP | 2020-528740 A | 01 October 2020 |
| | | JP | 2020-530931 A | 29 October 2020 |
| | | JP | 2020-532995 A | 19 November 2020 |
| | | JP | 2020-533998 A | 26 November 2020 |
| | | JP | 2020-535121 A | 03 December 2020 |
| | | JP | 2021-502124 A | 28 January 2021 |
| | | JP | 2021-514606 A | 17 June 2021 |
| | | KR | 10-2019-0033561 A | 29 March 2019 |
| | | KR | 10-2019-0055127 A | 22 May 2019 |
| | | KR | 10-2020-0045557 A | 04 May 2020 |
| | | KR | 10-2020-0047686 A | 07 May 2020 |
| | | KR | 10-2020-0049861 A | 08 May 2020 |
| | | KR | 10-2020-0054203 A | 19 May 2020 |
| | | KR | 10-2020-0059208 A | 28 May 2020 |
| | | KR | 10-2020-0096230 A | 11 August 2020 |
| | | KR | 10-2020-0128558 A | 13 November 2020 |
| | | US | 10073952 B2 | 11 September 2018 |
| | | US | 10169541 B2 | 01 January 2019 |
| | | US | 10242160 B2 | 26 March 2019 |
| | | US | 10246753 B2 | 02 April 2019 |
| | | US | 10265009 B2 | 23 April 2019 |
| | | US | 10268803 B2 | 23 April 2019 |
| | | US | 10282520 B2 | 07 May 2019 |
| | | US | 10289805 B2 | 14 May 2019 |
| | | US | 10290374 B2 | 14 May 2019 |
| | | US | 10290375 B2 | 14 May 2019 |
| | | US | 10290376 B2 | 14 May 2019 |
| | | US | 10297351 B2 | 21 May 2019 |
| | | US | 10311973 B2 | 04 June 2019 |
| | | US | 10325071 B2 | 18 June 2019 |
| | | US | 10325683 B2 | 18 June 2019 |
| | | US | 10325684 B2 | 18 June 2019 |
| | | US | 10325685 B2 | 18 June 2019 |
| | | US | 10327641 B2 | 25 June 2019 |
| | | US | 10327642 B2 | 25 June 2019 |
| | | US | 10331857 B2 | 25 June 2019 |
| | | US | 10332635 B2 | 25 June 2019 |
| | | US | 10340045 B2 | 02 July 2019 |
| | | US | 10346588 B2 | 09 July 2019 |
| | | US | 10346589 B2 | 09 July 2019 |
| | | US | 10346592 B2 | 09 July 2019 |
| | | US | 10347362 B2 | 09 July 2019 |

International application No.

**PCT/KR2021/007567**

Form PCT/ISA/210 (patent family annex) (July 2019)

| Patent document cited in search report | Publication date (day/month/year) | Patent family member(s) | | | Publication date (day/month/year) |
|---|---|---|---|---|---|
| | | US | 10347366 | B2 | 09 July 2019 |
| | | US | 10347367 | B2 | 09 July 2019 |
| | | US | 10347368 | B2 | 09 July 2019 |
| | | US | 10347379 | B2 | 09 July 2019 |
| | | US | 10354756 | B2 | 16 July 2019 |
| | | US | 10354757 | B2 | 16 July 2019 |
| | | US | 10357157 | B2 | 23 July 2019 |
| | | US | 10358682 | B2 | 23 July 2019 |
| | | US | 10360346 | B2 | 23 July 2019 |
| | | US | 10360347 | B2 | 23 July 2019 |
| | | US | 10360348 | B2 | 23 July 2019 |
| | | US | 10366782 | B2 | 30 July 2019 |
| | | US | 10366789 | B2 | 30 July 2019 |
| | | US | 10366793 | B2 | 30 July 2019 |
| | | US | 10380325 | B2 | 13 August 2019 |
| | | US | 10381112 | B2 | 13 August 2019 |
| | | US | 10381117 | B2 | 13 August 2019 |
| | | US | 10383519 | B2 | 20 August 2019 |
| | | US | 10388407 | B2 | 20 August 2019 |
| | | US | 10395777 | B2 | 27 August 2019 |
| | | US | 10409955 | B2 | 10 September 2019 |
| | | US | 10410749 | B2 | 10 September 2019 |
| | | US | 10755800 | B2 | 25 August 2020 |
| | | US | 10777320 | B2 | 15 September 2020 |
| | | US | 10786194 | B2 | 29 September 2020 |
| | | US | 10786195 | B2 | 29 September 2020 |
| | | US | 10787714 | B2 | 29 September 2020 |
| | | US | 10789334 | B2 | 29 September 2020 |
| | | US | 10790042 | B2 | 29 September 2020 |
| | | US | 10790043 | B2 | 29 September 2020 |
| | | US | 10790060 | B2 | 29 September 2020 |
| | | US | 10790061 | B2 | 29 September 2020 |
| | | US | 10793907 | B2 | 06 October 2020 |
| | | US | 10795970 | B2 | 06 October 2020 |
| | | US | 10795971 | B2 | 06 October 2020 |
| | | US | 10795972 | B2 | 06 October 2020 |
| | | US | 10796783 | B2 | 06 October 2020 |
| | | US | 10796785 | B2 | 06 October 2020 |
| | | US | 10796786 | B2 | 06 October 2020 |
| | | US | 10796800 | B2 | 06 October 2020 |
| | | US | 10803147 | B2 | 13 October 2020 |
| | | US | 10803991 | B2 | 13 October 2020 |
| | | US | 10803992 | B2 | 13 October 2020 |
| | | US | 10902938 | B2 | 26 January 2021 |
| | | US | 2016-0110515 | A1 | 21 April 2016 |
| | | US | 2016-0224748 | A1 | 04 August 2016 |
| | | US | 2016-0224749 | A1 | 04 August 2016 |
| | | US | 2016-0228003 | A1 | 11 August 2016 |
| | | US | 2016-0230217 | A1 | 11 August 2016 |
| | | US | 2016-0232280 | A1 | 11 August 2016 |

Form PCT/ISA/210 (patent family annex) (July 2019)

INTERNATIONAL SEARCH REPORT
Information on patent family members

International application No.

**PCT/KR2021/007567**

| Patent document cited in search report | Publication date (day/month/year) | Patent family member(s) | | Publication date (day/month/year) |
|---|---|---|---|---|
| | | US 2016-0232312 | A1 | 11 August 2016 |
| | | US 2016-0232313 | A1 | 11 August 2016 |
| | | US 2016-0232319 | A1 | 11 August 2016 |
| | | US 2016-0259909 | A1 | 08 September 2016 |
| | | US 2016-0314281 | A1 | 27 October 2016 |
| | | US 2016-0319358 | A1 | 03 November 2016 |
| | | US 2016-0321416 | A1 | 03 November 2016 |
| | | US 2016-0342735 | A1 | 24 November 2016 |
| | | US 2017-0024527 | A1 | 26 January 2017 |
| | | US 2017-0035346 | A1 | 09 February 2017 |
| | | US 2017-0039347 | A1 | 09 February 2017 |
| | | US 2017-0053061 | A1 | 23 February 2017 |
| | | US 2017-0175172 | A1 | 22 June 2017 |
| | | US 2017-0228514 | A1 | 10 August 2017 |
| | | US 2017-0235902 | A1 | 17 August 2017 |
| | | US 2017-0262608 | A1 | 14 September 2017 |
| | | US 2017-0270268 | A1 | 21 September 2017 |
| | | US 2017-0270269 | A1 | 21 September 2017 |
| | | US 2017-0270270 | A1 | 21 September 2017 |
| | | US 2017-0270271 | A1 | 21 September 2017 |
| | | US 2017-0270272 | A1 | 21 September 2017 |
| | | US 2017-0277843 | A1 | 28 September 2017 |
| | | US 2017-0277844 | A1 | 28 September 2017 |
| | | US 2017-0277845 | A1 | 28 September 2017 |
| | | US 2017-0277846 | A1 | 28 September 2017 |
| | | US 2017-0277847 | A1 | 28 September 2017 |
| | | US 2017-0277848 | A1 | 28 September 2017 |
| | | US 2017-0286612 | A1 | 05 October 2017 |
| | | US 2017-0286619 | A1 | 05 October 2017 |
| | | US 2017-0286620 | A1 | 05 October 2017 |
| | | US 2017-0298418 | A1 | 19 October 2017 |
| | | US 2017-0308657 | A1 | 26 October 2017 |
| | | US 2017-0308658 | A1 | 26 October 2017 |
| | | US 2017-0308659 | A1 | 26 October 2017 |
| | | US 2017-0308660 | A1 | 26 October 2017 |
| | | US 2017-0308669 | A1 | 26 October 2017 |
| | | US 2017-0316151 | A1 | 02 November 2017 |
| | | US 2017-0316152 | A1 | 02 November 2017 |
| | | US 2017-0316166 | A1 | 02 November 2017 |
| | | US 2017-0316167 | A1 | 02 November 2017 |
| | | US 2017-0316168 | A1 | 02 November 2017 |
| | | US 2017-0316169 | A1 | 02 November 2017 |
| | | US 2017-0327863 | A1 | 16 November 2017 |
| | | US 2017-0327864 | A1 | 16 November 2017 |
| | | US 2017-0329895 | A1 | 16 November 2017 |
| | | US 2017-0329896 | A1 | 16 November 2017 |
| | | US 2017-0329897 | A1 | 16 November 2017 |
| | | US 2017-0329898 | A1 | 16 November 2017 |
| | | US 2017-0329923 | A1 | 16 November 2017 |
| | | US 2017-0344719 | A1 | 30 November 2017 |

Form PCT/ISA/210 (patent family annex) (July 2019)

INTERNATIONAL SEARCH REPORT
Information on patent family members

| International application No. |
|---|
| **PCT/KR2021/007567** |

| Patent document cited in search report | Publication date (day/month/year) | Patent family member(s) | | Publication date (day/month/year) |
|---|---|---|---|---|
| | | US | 2017-0357762 A1 | 14 December 2017 |
| | | US | 2017-0357763 A1 | 14 December 2017 |
| | | US | 2017-0357767 A1 | 14 December 2017 |
| | | US | 2017-0357768 A1 | 14 December 2017 |
| | | US | 2017-0367640 A1 | 28 December 2017 |
| | | US | 2017-0367641 A1 | 28 December 2017 |
| | | US | 2017-0369933 A1 | 28 December 2017 |
| | | US | 2017-0372027 A1 | 28 December 2017 |
| | | US | 2018-0070825 A1 | 15 March 2018 |
| | | US | 2018-0070826 A1 | 15 March 2018 |
| | | US | 2018-0070827 A1 | 15 March 2018 |
| | | US | 2018-0090552 A1 | 29 March 2018 |
| | | US | 2018-0102187 A1 | 12 April 2018 |
| | | US | 2018-0114592 A1 | 26 April 2018 |
| | | US | 2018-0122510 A1 | 03 May 2018 |
| | | US | 2018-0122511 A1 | 03 May 2018 |
| | | US | 2018-0122512 A1 | 03 May 2018 |
| | | US | 2018-0122513 A1 | 03 May 2018 |
| | | US | 2018-0122514 A1 | 03 May 2018 |
| | | US | 2018-0122515 A1 | 03 May 2018 |
| | | US | 2018-0122887 A1 | 03 May 2018 |
| | | US | 2018-0137239 A1 | 17 May 2018 |
| | | US | 2018-0137930 A1 | 17 May 2018 |
| | | US | 2018-0137931 A1 | 17 May 2018 |
| | | US | 2018-0286520 A1 | 04 October 2018 |
| | | US | 2018-0342322 A1 | 29 November 2018 |
| | | US | 2018-0362967 A1 | 20 December 2018 |
| | | US | 2019-0019575 A1 | 17 January 2019 |
| | | US | 2019-0050534 A1 | 14 February 2019 |
| | | US | 2019-0078085 A1 | 14 March 2019 |
| | | US | 2019-0080046 A1 | 14 March 2019 |
| | | US | 2019-0085396 A1 | 21 March 2019 |
| | | US | 2019-0136288 A1 | 09 May 2019 |
| | | US | 2020-0123539 A1 | 23 April 2020 |
| | | US | 2020-0202979 A1 | 25 June 2020 |
| | | US | 2020-0263171 A1 | 20 August 2020 |
| | | US | 2020-0286623 A1 | 10 September 2020 |
| | | US | 2020-0303070 A1 | 24 September 2020 |
| | | US | 2020-0392481 A1 | 17 December 2020 |
| | | US | 9703929 B2 | 11 July 2017 |
| | | US | 9710606 B2 | 18 July 2017 |
| | | US | 9754080 B2 | 05 September 2017 |
| | | US | 9758839 B2 | 12 September 2017 |
| | | US | 9760676 B2 | 12 September 2017 |
| | | WO | 2016-065075 A1 | 28 April 2016 |
| | | WO | 2016-168316 A1 | 20 October 2016 |
| | | WO | 2016-168336 A1 | 20 October 2016 |
| | | WO | 2016-168344 A1 | 20 October 2016 |
| | | WO | 2016-168350 A1 | 20 October 2016 |
| | | WO | 2016-168352 A1 | 20 October 2016 |

Form PCT/ISA/210 (patent family annex) (July 2019)

International application No.

**PCT/KR2021/007567**

| Patent document cited in search report | | | Publication date (day/month/year) | Patent family member(s) | | | Publication date (day/month/year) |
|---|---|---|---|---|---|---|---|
| | | | | WO | 2016-168354 | A1 | 20 October 2016 |
| | | | | WO | 2016-168355 | A1 | 20 October 2016 |
| | | | | WO | 2016-168359 | A1 | 20 October 2016 |
| | | | | WO | 2016-168364 | A1 | 20 October 2016 |
| | | | | WO | 2016-168370 | A1 | 20 October 2016 |
| | | | | WO | 2017-044901 | A1 | 16 March 2017 |
| | | | | WO | 2017-100688 | A1 | 15 June 2017 |
| | | | | WO | 2017-156031 | A1 | 14 September 2017 |
| | | | | WO | 2017-189614 | A1 | 02 November 2017 |
| | | | | WO | 2018-013865 | A1 | 18 January 2018 |
| | | | | WO | 2018-053443 | A1 | 22 March 2018 |
| | | | | WO | 2018-094376 | A1 | 24 May 2018 |
| | | | | WO | 2018-112419 | A1 | 21 June 2018 |
| | | | | WO | 2018-112437 | A1 | 21 June 2018 |
| | | | | WO | 2018-112459 | A1 | 21 June 2018 |
| | | | | WO | 2018-223146 | A1 | 06 December 2018 |
| | | | | WO | 2018-237092 | A1 | 27 December 2018 |
| | | | | WO | 2019-018580 | A1 | 24 January 2019 |
| | | | | WO | 2019-036176 | A1 | 21 February 2019 |
| | | | | WO | 2019-051130 | A1 | 14 March 2019 |
| | | | | WO | 2019-051319 | A1 | 14 March 2019 |
| | | | | WO | 2019-055874 | A1 | 21 March 2019 |
| | | | | WO | 2019-090353 | A1 | 09 May 2019 |
| | | | | WO | 2019-169395 | A1 | 06 September 2019 |
| KR | 10-2016-0069519 | A | 16 June 2016 | CN | 105829589 | A | 03 August 2016 |
| | | | | CN | 105829589 | B | 02 February 2021 |
| | | | | CN | 112877472 | A | 01 June 2021 |
| | | | | EP | 3066236 | A1 | 14 September 2016 |
| | | | | JP | 2017-500015 | A | 05 January 2017 |
| | | | | JP | 2021-072777 | A | 13 May 2021 |
| | | | | KR | 10-2021-0008941 | A | 25 January 2021 |
| | | | | US | 10450620 | B2 | 22 October 2019 |
| | | | | US | 2015-0133391 | A1 | 14 May 2015 |
| | | | | US | 2020-0017926 | A1 | 16 January 2020 |
| | | | | WO | 2015-070086 | A1 | 14 May 2015 |
| KR | 10-2020-0061403 | A | 02 June 2020 | AU | 2020-347619 | A1 | 09 April 2020 |
| | | | | CA | 3076359 | A1 | 18 April 2019 |
| | | | | CN | 111247254 | A | 05 June 2020 |
| | | | | EP | 3695017 | A1 | 19 August 2020 |
| | | | | JP | 2020-536548 | A | 17 December 2020 |
| | | | | US | 2020-0263223 | A1 | 20 August 2020 |
| | | | | WO | 2019-075426 | A1 | 18 April 2019 |
| WO | 2020-037271 | A1 | 20 February 2020 | AU | 2021-321681 | A1 | 25 February 2021 |
| | | | | CN | 112888447 | A | 01 June 2021 |
| | | | | EP | 3836946 | A1 | 23 June 2021 |
| | | | | KR | 10-2021-0091119 | A | 21 July 2021 |

Form PCT/ISA/210 (patent family annex) (July 2019)